# EUROPEAN PATENT SPECIFICATION

(11) **EP 2 964 244 B1**
(45) Date of publication and mention of the grant of the patent: **08.01.2020**
(21) Application number: 14713268.2
(22) Date of filing: 10.03.2014
(51) Int. Cl.: A61K 38/00, A61K 9/14, A61K 31/713, C08G 73/02

(54) **MODIFIED POLY(BETA-AMINO ESTER)S FOR DRUG DELIVERY**
MODIFIZIERTE POLY(BETA-AMINOESTER) ZUR ARZNEIMITTELABGABE
POLY(BÊTA-AMINO-ESTERS) MODIFIÉS POUR L'ADMINISTRATION D'UN MÉDICAMENT

(30) Priority: 08.03.2013 GB 201304245
(43) Date of publication of application: 13.01.2016
(73) Proprietor: Institut Químic de Sarrià CETS Fundació Privada, 08017 Barcelona (ES)
(72) Inventor: BORRÓS GÓMEZ, Salvador, E-08017 Barcelona (ES); RAMOS PÉREZ, Victor, E-08017 Barcelona (ES); SEGOVIA RAMOS, Nathaly, E-08017 Barcelona (ES); DOSTA PONS, Pere, E-08017 Barcelona (ES)
(74) Representative: Carpmaels & Ransford LLP
(86) International application number: PCT/IB2014/059594
(87) International publication number: WO 2014/136100

(56) References cited:
- WO-A2-2008/011561
- US-A1- 2012 114 759
- NATHALY SEGOVIA ET AL: "Oligopeptide-terminated poly([beta]-amino ester)s for highly efficient gene delivery and intracellular localization", ACTA BIOMATERIALIA, vol. 10, no. 5, 1 May 2014 (2014-05-01), pages 2147-2158, XP055119794, ISSN: 1742-7061, DOI: 10.1016/j.actbio.2013.12.054
- HYE SUN PARK ET AL: "pH-stimuli-responsive near-infrared optical imaging nanoprobe based on poly(-glutamic acid)/poly(-amino ester) nanoparticles;pH-stimuli-responsive near-infrared optical imaging nanoprobe based on poly(gamma-glutamic acid)/poly(beta-amino ester) nanoparticles", NANOTECHNOLOGY, IOP, BRISTOL, GB, vol. 22, no. 46, 27 October 2011 (2011-10-27), page 465603, XP020213382, ISSN: 0957-4484, DOI: 10.1088/0957-4484/22/46/465603

## Description

The invention relates to polymers suitable for use in delivery of active agents. The invention also pertains to nanoparticles comprising these polymers and methods for their production.

The lack of safe and efficient vectors to deliver polynucleotides such as DNA and RNA remains the principal handicap for the success of gene therapy (Luo, D. & Saltzman, W.M. Synthetic DNA delivery systems. Nature Biotech. 18, 33-37 (2000); Kamimura K. et al, Advances in Gene Delivery Systems. Pharmaceut. Med. 25, 293-306 (2011); Miele E. et al, Nanoparticle-based delivery of small interfering RNA: challenges for cancer therapy. Int. J. Nanomedicine 7, 3637-3657 (2012)). The majority of protocols for polynucleotide delivery employ viral vectors, which are highly efficient delivery systems. However, viral vectors have certain disadvantages, including safety risk, limited capacity to carry polynucleotides and high cost of large-scale production. Non-viral vectors offer potential advantages, including high packing capacity, ease of production, low toxicity and immunogenicity, but are less efficient than viral vectors (Mintzer, M. A. & Simanek, E.E. Nonviral vectors for gene delivery. Chem. Rev. 109, 259-302 (2009)).

Biodegradable poly(β-amino ester)s (PBAEs) have been described as potential non-viral polynucleotide delivery vectors capable of condensing both DNA and RNA into discrete nanometric particles (Green, J.J. et al. Acc. Chem Res. 41, 749-759 (2008)). Chemical modification at the termini of PBAEs with primary amines (see Scheme 1) has been shown to produce higher transfection efficacy than commercial transfection agents such as Lipofectamine 2000, Fugene and polyethylenimine (PEI) (Zugates, G.T. et al. Bioconjugate Chem. 18, 1887-1896 (2007); Green, J.J. et al. Nano letters 8, 3126-3130 (2008); WO02/31025A2).

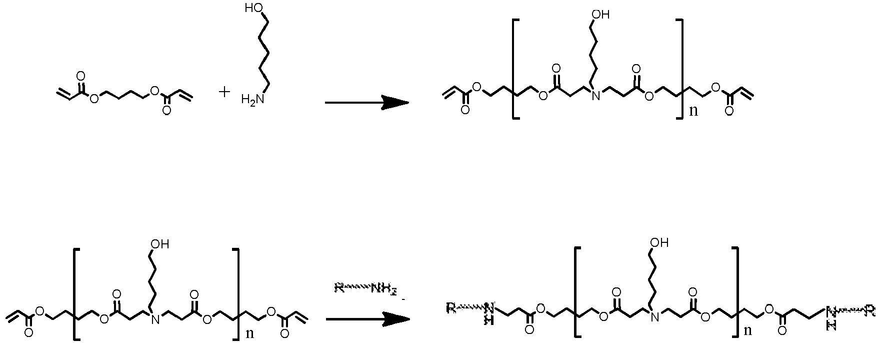

US 2012114759 A1 describes polymeric nanoparticles, microparticles, and gels for delivering cargo, e.g. a therapeutic agent, to a target.

A continuing need exists for improved non-toxic, biodegradable, biocompatible polymers that can be used to transfect polynucleotides efficiently and that can be prepared economically. Such polymers would be useful in the packaging and delivery of DNA and RNA in gene therapy and for the packaging and delivery of other diagnostic, therapeutic and prophylactic agents.

In particular, there is a need for polymers that can be used to efficiently transfect short polynucleotides, particularly siRNA and microRNA (miRNA), which has poor stability in circulation. Existing polymeric polynucleotide delivery vectors cannot encapsulate siRNA and miRNA with high loading owing to the relatively short length of these sequences. In addition, many existing polymeric delivery vectors for siRNA and miRNA are cytotoxic.

The present invention provides novel end-modified PBAEs useful in a variety of medical applications including drug delivery, particularly in the delivery of polynucleotides; tissue engineering and biomaterials. The present invention is particularly directed to medical applications of PBAEs. The invention also provides complexes of the inventive end-modified polymers with polynucleotides, drug delivery devices (e.g., microparticles, nanoparticles) including the inventive polymers, methods of preparing end-modified polymers, and methods of using the inventive end-modified polymers.

The polyester nature of these systems results in an attractive biocompatible profile owing to their high biodegradability and reduced toxicity. Therefore, these polymers have applications as non-viral polynucleotide delivery vectors in the treatment of many diseases such as cancer, monogenetic diseases, vascular disease and infectious diseases. Another application of these polynucleotide delivery vectors is *in vitro* research as a tool to investigate gene function or regulation within a cellular and physiological context.

In a first aspect, the invention provides polymers of **Formula I:** 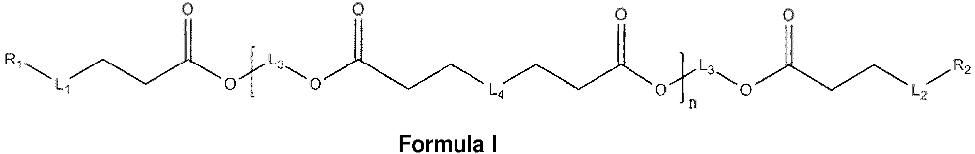 wherein
L₁ and L₂ are independently selected from the group consisting of: 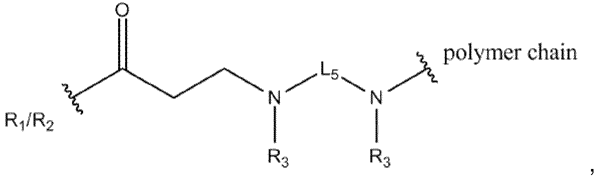
O, S, NRₓ and a bond; wherein Rₓ is independently selected from the group consisting of hydrogen, halogen, alkyl, cycloalkyl, alkenyl, cycloalkenyl, heteroalkyl, heterocycloalkyl, acyl, aryl or heteroaryl;
L₃ is independently selected from the group consisting of alkylene, alkenylene, heteroalkylene, heteroalkenylene, arylene or heteroarylene;
L₄ is independently selected from the group consisting of 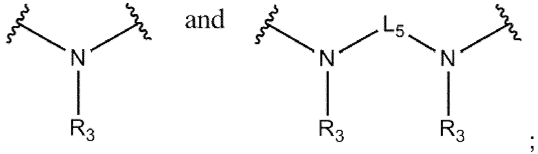
L₅ is independently selected from the group consisting of alkylene, alkenylene, heteroalkylene, heteroalkenylene, arylene or heteroarylene;
R₁ and R₂ are independently selected from an oligopeptide and R_{y};
wherein at least one of R₁ and R₂ is an oligopeptide; wherein the or each oligopeptide has a net positive charge at pH 7; or wherein the or each oligopeptide comprises a mixture of naturally occurring amino acids that are negatively charged at pH 7 and naturally occurring amino acids that are positively charged at pH 7;
and wherein R_{y} is selected from the group consisting of hydrogen, halogen, alkyl, cycloalkyl, alkenyl, cycloalkenyl, heteroalkyl, heterocycloalkyl, acyl, aryl or heteroaryl;
each R₃ is independently selected from the group consisting of hydrogen, halogen, alkyl, cycloalkyl, alkenyl, cycloalkenyl, heteroalkyl, heterocycloalkyl, acyl, aryl or heteroaryl; and n is an integer from 5 to 10,000;
or a pharmaceutically acceptable salt thereof.

In a second aspect of the disclosure is provided polymers of **Formula I**, wherein
L₁ and L₂ are independently selected from the group consisting of: 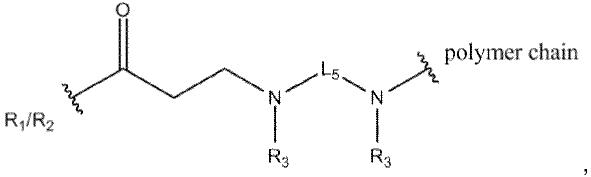 O, S, NRₓ and a bond; wherein Rₓ is independently selected from the group consisting of hydrogen, halogen, alkyl, cycloalkyl, alkenyl, cycloalkenyl, heteroalkyl, heterocycloalkyl, acyl, aryl or heteroaryl; at least one occurrence of L₃ is 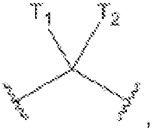 wherein T₁ is 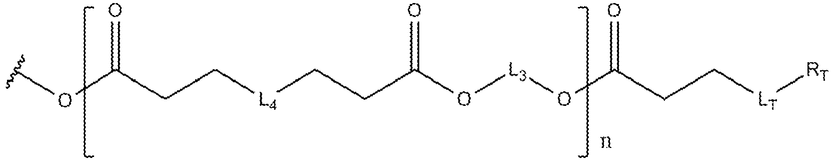 and T₂ is selected from H, alkyl or 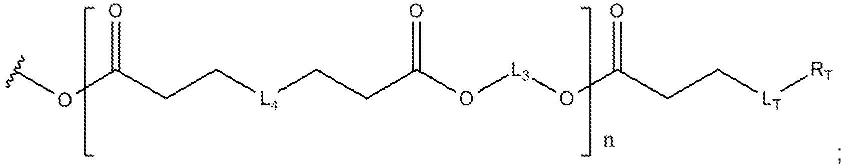 wherein L_{T} is independently selected from the group consisting of: 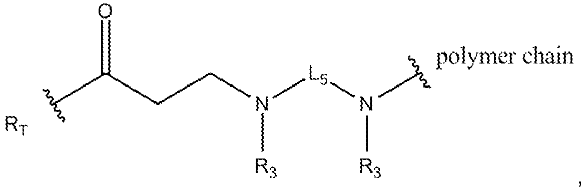
O, S, NRₓ and a bond; wherein Rₓ is independently selected from the group consisting of hydrogen, halogen, alkyl, cycloalkyl, alkenyl, cycloalkenyl, heteroalkyl, heterocycloalkyl, acyl, aryl or heteroaryl;
the remaining L₃ groups are independently selected at each occurrence from the group consisting of alkylene, alkenylene, heteroalkylene, heteroalkenylene, arylene or heteroarylene, wherein L₄ is independently selected from the group consisting of

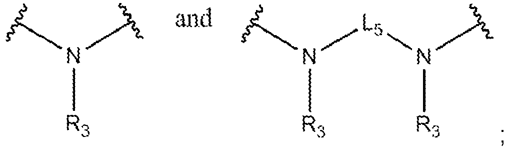

L₅ is independently selected from the group consisting of alkylene, alkenylene, heteroalkylene, heteroalkenylene, arylene or heteroarylene;
R₁, R₂ and R_{T} are independently selected from an oligopeptide and R_{y};
wherein at least one of R_{1,} R₂ and R_{T} is an oligopeptide;
and wherein R_{y} is selected from the group consisting of hydrogen, halogen, alkyl, cycloalkyl, alkenyl, cycloalkenyl, heteroalkyl, heterocycloalkyl, acyl, aryl or heteroaryl;
each R₃ is independently selected from the group consisting of hydrogen, halogen, alkyl, cycloalkyl, alkenyl, cycloalkenyl, heteroalkyl, heterocycloalkyl, acyl, aryl or heteroaryl; and
n is an integer from 5 to 10,000;
or a pharmaceutically acceptable salt thereof.

The present invention thus provides PBAEs end-modified with at least one oligopeptide. These PBAEs act as superior condensing agents for polynucleotides and/or as targeting ligands to enhance cellular uptake and transfection efficiency. These polymers have biodegradable groups capable of improving the delivery of polynucleotides to cells and have shown high transfection efficacy and reduced cytotoxicity in vitro compared with known PBAEs and commercial transfection agents.

The polymers of Formula I may be prepared by the reaction of diacrylate monomers of **Formula II** with substituted amines of formula L₄H₂ to form an acrylate terminated intermediate, **Formula III.**

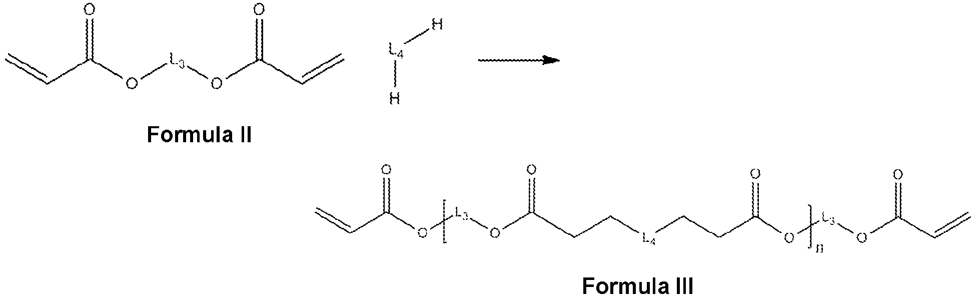

Groups R₁L₁ and R₂L₂ may then be added by reaction with a terminal acrylate group to form a polymer of Formula I.

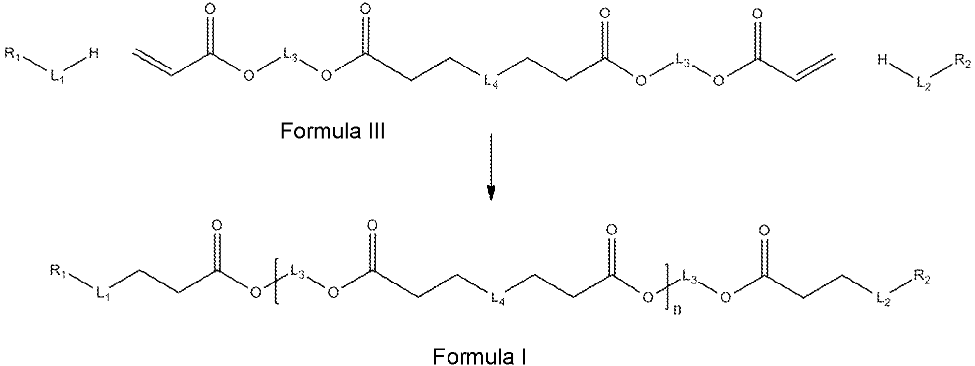

In polymers according to the present invention, each L₁ and L₂ (and, in the second aspect, L_{T}) is selected to facilitate coupling of the end-modifying groups R₁ and R₂ to the PBAE polymer. Each L₁ and L₂ (and, in the second aspect, L_{T}) may be a bond, for example where the end-modifying group is an oligopeptide that comprises a terminal cysteine residue.

In the second aspect of the disclosure, L_{T} is selected to facilitate coupling of the end-modifying group R_{T} to the PBAE polymer. L_{T} may be a bond, for example where the end-modifying group is an oligopeptide that comprises a terminal cysteine residue.

In polymers according to the present invention, Rₓ may be independently selected from the group consisting of hydrogen, alkyl, cycloalkyl, alkenyl, cycloalkenyl, heteroalkyl and heterocycloalkyl, for example, from the group consisting of hydrogen, alkyl and cycloalkyl.

According to the present invention, an "oligopeptide" comprises a string of at least three amino acids linked together by peptide bonds. Such peptides preferably contain only natural amino acids, although non-natural amino acids (i.e., compounds that do not occur in nature but that can be incorporated into a polypeptide chain) and/or amino acid analogues as are known in the art may alternatively be employed. Also, one or more of the amino acids in such peptides may be modified, for example, by the addition of a chemical entity such as a carbohydrate group, a phosphate group, a farnesyl group, an isofarnesyl group, a fatty acid group, or a linker for conjugation, functionalization, or other modification, etc. The oligopeptides in the polymers of the present invention typically comprise from 3 to 20 amino acid residues, more preferably from 3 to 10 amino acid residues, more preferably from 3 to 6 amino acid residues. Alternatively, the oligopeptides in the polymers of the present invention may comprise from 4 to 20 amino acid residues, more preferably from 4 to 10 amino acid residues, more preferably from 4 to 6 amino acid residues.

The present invention further provides polymers of formula I wherein the or each oligopeptide has a net positive charge at pH7. The or each oligopeptide may comprise naturally occurring amino acids that are positively charged at pH7, that is, lysine, arginine and histidine. For example, the or each oligopeptide may be selected from the group consisting of polylysine, polyarginine or polyhistidine, each of which may be terminated with cysteine.

In an embodiment, the or each oligopeptide is preferably a compound of Formula IV: 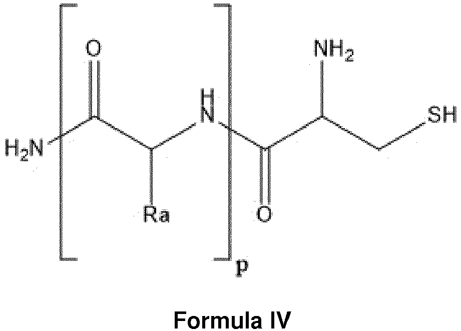 wherein p is an integer from 2 to 19, typically from 3 to 9 or from 3 to 5, and wherein Rₐ is selected at each occurrence from the group consisting of H2NC(=NH)-NH(CH2)3-, H₂N(CH₂)₄- or (1*H*-imidazol-4-yl)-CH₂-.

Where the or each oligopeptide is a compound of Formula IV the L₁ and/or L₂ (and/or, in the second aspect, L_{T}) linking the or each oligopeptide to the polymer is a bond and the terminal cysteine residue provides a means of coupling the or each oligopeptide to the acrylate terminated intermediate, Formula III. The thiol functionality provides faster, more efficient and more easily controlled addition to the double bond. By contrast, where the or each oligopeptide is terminated in an amine functionality for coupling, an excess of this compound is required in the coupling step.

The present disclosure further provides polymers of formula I wherein the or each oligopeptide has a net negative charge at pH7. The or each oligopeptide may comprise naturally occurring amino acids that are negatively charged at pH7, that is, aspartic acid and glutamic acid. For example, the or each oligopeptide may be selected from the group consisting of polyaspartic acid and polyglutamic acid, each of which may be terminated with cysteine. In this embodiment, the or each oligopeptide may be a compound of Formula IV wherein p is an integer from 2 to 19, typically from 3 to 9 or from 3 to 5, and wherein Rₐ is HO₂C(CH₂)₂- or HO₂C-CH₂-. In this case, the L₁ and/or L₂ linking the or each oligopeptide to the polymer is a bond as the terminal cysteine residue provides a means of coupling the or each oligopeptide to the acrylate terminated intermediate, formula IV.

Alternatively in the present invention, the or each oligopeptide may comprise a mixture of naturally occurring amino acids that are negatively charged at pH7 and naturally occurring amino acids that are positively charged at pH7.

The present invention further provides polymers of formula I wherein the or each oligopeptide is hydrophobic. The or each oligopeptide may comprise naturally occurring amino acids that are hydrophobic such as valine, leucine, isoleucine, methionine, tryptophan, phenylalanine, cysteine, tyrosine and alanine; in particular, the or each oligopeptide may comprise valine, leucine, isoleucine, methionine, tryptophan and phenylalanine.

The present invention further provides polymers of formula I wherein the or each oligopeptide is hydrophilic. The or each oligopeptide may comprise naturally occurring amino acids that are hydrophilic such as serine, threonine, cysteine, asparagine and glutamine, and may further comprise naturally occurring amino acids that are charged at pH7.

According to the first aspect of the invention there are provided polymers of formula I wherein both R₁ and R₂ are oligopeptides and polymers of formula I wherein one of R₁ and R₂ is an oligopeptide and one of R₁ and R₂ is R_{y}.

According to the first aspect of the invention, where one of R₁ and R₂ is R_{y}, then R_{y} is preferably selected from the group consisting of hydrogen, -(CH₂)ₘNH₂, -(CH₂)ₘNHMe, - (CH₂)ₘOH, -(CH₂)ₘCH₃, -(CH₂)₂(OCH₂CH₂)ₘNH₂, -(CH₂)₂(OCH₂CH₂)ₘOH and - (CH₂)₂(OCH₂CH₂)ₘCH₃ wherein m is an integer from 1 to 20, for example from 1 to 5. Preferably, R_{y} is selected from the group consisting of -(CH₂)ₘNH₂, -(CH₂)ₘNHMe and - (CH₂)₂(OCH₂CH₂)ₘNH₂. Preferably, when L₁ is NH or NRₓ, and one of R₁ and R₂ is R_{y}, then R_{y} is different to R₃.

The polymers of the present invention may be asymmetric. For example, in polymers according to the first aspect of the invention one of R₁ and R₂ may be an oligopeptide and the other may be R_{y}. Alternatively, R₁ and R₂ may each be a different oligopeptide. In polymers according to the second aspect of the disclosure at least one selected from R₁, R₂ and the one or two occurrences of R₅ may be an oligopeptide and the remaining groups selected from R₁, R₂ and the one or two occurrences of R_{T} may be R_{y}. Alternatively, R₁, R₂ and the one or two occurrences of R_{T} may each be a different oligopeptide.

The inventors have found that asymmetric polymers have higher polynucleotide delivery efficiency. For example, polymers according to the first aspect of the invention wherein one of R₁ and R₂ is CysArgArgArg and the other derived from H₂N(CH₂)₃CH(CH₃)CH₂NH₂ have higher polynucleotide delivery efficiency than both polymers in which both R₁ and R₂ are CysArgArgArg, and polymers wherein both R₁ and R₂ are derived from H₂N(CH₂)₃CH(CH₃)CH₂NH₂.

In polymers according to the present invention, L₃ and L₅ may be independently selected from alkylene, alkenylene, heteroalkylene or heteroalkenylene and polyethylene glycol linkers. Said alkylene, alkenylene, heteroalkylene or heteroalkenylene moieties may be of 1-20 carbon atoms, preferably of 1-12 carbon atoms, more preferably of 1-6 carbon atoms. Said polyethylene glycol linkers may be of 3 to 25 atoms in length, preferably of 3 to 18 atoms in length.

Optionally, one or more carbon atoms in L₃ and/or L₅ may be replaced with -S-S-. The inclusion of at least one disulfide bond in the main polymer chain allows efficient unpacking of therapeutic polynucleotides inside the target cells.

In polymers according to the present invention, each R₃ may be independently selected from the group consisting of hydrogen, -(CH₂)ₚNH₂, -(CH₂)ₚNHMe, -(CH₂)ₚOH, -(CH₂)ₚCH₃, - (CH₂)₂(OCH₂CH₂)_{q}NH₂, -(CH₂)₂(OCH₂CH₂)_{q}OH and -(CH₂)₂(OCH₂CH₂)_{q}CH₃ wherein p is an integer from 1 to 20, for example from 1 to 5, and q is an integer from 1 to 10, for example from 1 to 5.

In formula I or II above, n is preferably from 5 to 1000, more preferably from 20 to 500. The molecular weight of the polymer of formula I or formula II is preferably from 1,000 to 100,000 g/mol, more preferably 2,000 and 50,000 g/mol more preferably 5,000 and 40,000 g/mol.

Certain compounds of the present invention may exist in particular geometric or stereoisomeric forms. The present invention contemplates all such compounds, including cis- and trans-isomers, R- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

Isomeric mixtures containing any of a variety of isomer ratios may be utilized in accordance with the present invention. For example, where only two isomers are combined, mixtures containing 50:50, 60:40, 70:30, 80:20, 90: 10, 95:5, 96:4, 97:3, 98:2 or 99:1 isomer ratios are all contemplated by the present invention. Those of ordinary skill in the art will readily appreciate that analogous ratios are contemplated for more complex isomer mixtures.

### Chemical Groups

The term "halogen" (or "halo") includes fluorine, chlorine, bromine and iodine.

The term "alkyl" includes monovalent, straight or branched, saturated, acyclic hydrocarbyl groups. In one embodiment alkyl is C₁₋₁₀alkyl, in another embodiment C₁₋₆alkyl, in another embodiment C₁₋₄alkyl, such as methyl, ethyl, n-propyl, i-propyl or t-butyl groups. Alkyl may be substituted.

The term "cycloalkyl" includes monovalent, saturated, cyclic hydrocarbyl groups. In one embodiment cycloalkyl is C₃₋₁₀cycloalkyl, in another embodiment C₃₋₆cycloalkyl such as cyclopentyl and cyclohexyl. Cycloalkyl may be substituted.

The term "alkoxy" means alkyl-O-.

The term "alkylamino" means alkyl-NH-.

The term "alkylthio" means alkyl-S(O)ₜ-, wherein t is defined below.

The term "alkenyl" includes monovalent, straight or branched, unsaturated, acyclic hydrocarbyl groups having at least one carbon-carbon double bond and, in one embodiment, no carbon-carbon triple bonds. In one embodiment alkenyl is C₂₋₁₀alkenyl, in another embodiment C₂₋₆alkenyl, in another embodiment C₂₋₄alkenyl. Alkenyl may be substituted.

The term "cycloalkenyl" includes monovalent, partially unsaturated, cyclic hydrocarbyl groups having at least one carbon-carbon double bond and, in one embodiment, no carbon-carbon triple bonds. In one embodiment cycloalkenyl is C₃₋₁₀cycloalkenyl, in another embodiment C₅₋₁₀cycloalkenyl, *e.g*. cyclohexenyl or benzocyclohexyl. Cycloalkenyl may be substituted.

The term "alkynyl" includes monovalent, straight or branched, unsaturated, acyclic hydrocarbyl groups having at least one carbon-carbon triple bond and, in one embodiment, no carbon-carbon double bonds. In one embodiment, alkynyl is C₂₋₁₀alkynyl, in another embodiment C₂₋₆alkynyl, in another embodiment C₂₋₄alkynyl. Alkynyl may be substituted.

The term "alkylene" includes divalent, straight or branched, saturated, acyclic hydrocarbyl groups. In one embodiment alkylene is C₁₋₁₀alkylene, in another embodiment C₁₋₆alkylene, in another embodiment C₁₋₄alkylene, such as methylene, ethylene, n-propylene, i-propylene or t-butylene groups. Alkylene may be substituted.

The term "alkenylene" includes divalent, straight or branched, unsaturated, acyclic hydrocarbyl groups having at least one carbon-carbon double bond and, in one embodiment, no carbon-carbon triple bonds. In one embodiment alkenylene is C₂₋₁₀alkenylene, in another embodiment C₂₋₆alkenylene, in another embodiment C₂₋₄alkenylene. Alkenyene may be substituted.

The term "heteroalkyl" includes alkyl groups, for example, C₁₋₆₅alkyl groups, C₁₋₁₇alkyl groups or C₁₋₁₀alkyl groups, in which up to twenty carbon atoms, in an embodiment up to ten carbon atoms, in one embodiment up to two carbon atoms, in another embodiment one carbon atom, are each replaced independently by O, S(O)ₜ or N, provided at least one of the alkyl carbon atoms remains. The heteroalkyl group may be C-linked or hetero-linked, *i.e.* it may be linked to the remainder of the molecule through a carbon atom or through O, S(O)ₜ or N, wherein t is defined below. Heteroalkyl may be substituted.

The term "heterocycloalkyl" includes cycloalkyl groups in which up to ten carbon atoms, in one embodiment up to two carbon atoms, in another embodiment one carbon atom, are each replaced independently by O, S(O)ₜ or N, provided at least one of the cycloalkyl carbon atoms remains. Examples of heterocycloalkyl groups include oxiranyl, thiaranyl, aziridinyl, oxetanyl, thiatanyl, azetidinyl, tetrahydrofuranyl, tetrahydrothiophenyl, pyrrolidinyl, tetrahydropyranyl, tetrahydrothiopyranyl, piperidinyl, 1,4-dioxanyl, 1,4-oxathianyl, morpholinyl, 1,4-dithianyl, piperazinyl, 1,4-azathianyl, oxepanyl, thiepanyl, azepanyl, 1,4-dioxepanyl, 1,4-oxathiepanyl, 1,4-oxaazepanyl, 1,4-dithiepanyl, 1,4-thieazepanyl and 1,4-diazepanyl. The heterocycloalkyl group may be C-linked or N-linked, *i.e.* it may be linked to the remainder of the molecule through a carbon atom or through a nitrogen atom. Heterocycloalkyl may be substituted.

The term "heteroalkenyl" includes alkenyl groups, for example, C₁₋₆₅alkenyl groups, C₁₋₁₇alkenyl groups or C₁₋₁₀alkenyl groups, in which up to twenty carbon atoms, in an embodiment up to ten carbon atoms, in one embodiment up to two carbon atoms, in another embodiment one carbon atom, are each replaced independently by O, S(O)ₜ or N, provided at least one of the alkenyl carbon atoms remains. The heteroalkenyl group may be C-linked or hetero-linked, *i.e.* it may be linked to the remainder of the molecule through a carbon atom or through O, S(O)ₜ or N. Heteralkenyl may be substituted.

The term "heterocycloalkenyl" includes cycloalkenyl groups in which up to three carbon atoms, in one embodiment up to two carbon atoms, in another embodiment one carbon atom, are each replaced independently by O, S(O)ₜ or N, provided at least one of the cycloalkenyl carbon atoms remains. Examples of heterocycloalkenyl groups include 3,4-dihydro-2H-pyranyl, 5-6-dihydro-2H-pyranyl, 2H-pyranyl, 1,2,3,4-tetrahydropyridinyl and 1,2,5,6-tetrahydropyridinyl. The heterocycloalkenyl group may be C-linked or N-linked, *i.e.* it may be linked to the remainder of the molecule through a carbon atom or through a nitrogen atom. Heterocycloalkenyl may be substituted.

The term "heteroalkynyl" includes alkynyl groups, for example, C₁₋₆₅alkynyl groups, C₁₋₁₇alkynyl groups or C₁₋₁₀alkynyl groups, in which up to twenty carbon atoms, in an embodiment in which up to ten carbon atoms, in one embodiment up to two carbon atoms, in another embodiment one carbon atom, are each replaced independently by O, S(O)ₜ or N, provided at least one of the alkynyl carbon atoms remains. The heteroalkynyl group may be C-linked or hetero-linked, *i.e.* it may be linked to the remainder of the molecule through a carbon atom or through O, S(O)ₜ or N. Heteroalkynyl may be substituted.

The term "heteroalkylene" includes alkylene groups, for example, C₁₋₆₅alkylene groups, C₁₋₁₇alkylene groups or C₁₋₁₀alkylene groups, in which up to twenty carbon atoms, in an embodiment in which up to ten carbon atoms, in one embodiment up to two carbon atoms, in another embodiment one carbon atom, are each replaced independently by O, S(O)ₜ or N, provided at least one of the alkylene carbon atoms remains. Heteroalkynylene may be substituted.

The term "heteroalkenylene" includes alkenylene groups, for example, C₁₋₆₅alkenylene groups, C₁₋₁₇alkenylene groups or C₁₋₁₀alkenylene groups, in which up to twenty carbon atoms, in an embodiment in which up to ten carbon atoms, in one embodiment up to two carbon atoms, in another embodiment one carbon atom, are each replaced independently by O, S(O)ₜ or N, provided at least one of the alkenylene carbon atoms remains. Heteroalkenylene may be substituted.

The term "aryl" includes monovalent, aromatic, cyclic hydrocarbyl groups, such as phenyl or naphthyl (*e.g*. 1-naphthyl or 2-naphthyl). In general, the aryl groups may be monocyclic or polycyclic fused ring aromatic groups. Preferred aryl are C₆-C₁₄aryl. Aryl may be substituted.

Other examples of aryl groups are monovalent derivatives of aceanthrylene, acenaphthylene, acephenanthrylene, anthracene, azulene, chrysene, coronene, fluoranthene, fluorene, *as-*indacene, *s*-indacene, indene, naphthalene, ovalene, perylene, phenalene, phenanthrene, picene, pleiadene, pyrene, pyranthrene and rubicene.

The term "arylalkyl" means alkyl substituted with an aryl group, *e.g***.** benzyl.

The term "heteroaryl" includes aryl groups in which one or more carbon atoms are each replaced by heteroatoms independently selected from O, S, N and NR^{N}, where R^{N} is defined below (and in one embodiment is H or alkyl (*e.g*. C₁₋₆alkyl)). Heteroaryl may be substituted.

In general, the heteroaryl groups may be monocyclic or polycyclic (*e.g*. bicyclic) fused ring heteroaromatic groups. Typically, heteroaryl groups contain 5-14 ring members (preferably 5-10 members) wherein 1, 2, 3 or 4 ring members are independently selected from O, S, N and NR^{N}. In one embodiment, a heteroaryl group may be 5, 6, 9 or 10 membered, *e.g*. 5-membered monocyclic, 6-membered monocyclic, 9-membered fused-ring bicyclic or 10-membered fused-ring bicyclic.

Monocyclic heteroaromatic groups include heteroaromatic groups containing 5-6 ring members wherein 1, 2, 3 or 4 ring members are independently selected from O, S, N or NR^{N}.

In one embodiment, 5-membered monocyclic heteroaryl groups contain 1 ring member which is an -NR^{N}- group, an -O- atom or an -S- atom and, optionally, 1-3 ring members (e.g. 1 or 2 ring members) which are =N- atoms (where the remainder of the 5 ring members are carbon atoms).

Examples of 5-membered monocyclic heteroaryl groups are pyrrolyl, furanyl, thiophenyl, pyrazolyl, imidazolyl, isoxazolyl, oxazolyl, isothiazolyl, thiazolyl, 1,2,3 triazolyl, 1,2,4 triazolyl, 1,2,3 oxadiazolyl, 1,2,4 oxadiazolyl, 1,2,5 oxadiazolyl, 1,3,4 oxadiazolyl, 1,3,4 thiadiazolyl, pyridyl, pyrimidinyl, pyridazinyl, pyrazinyl, 1,3,5 triazinyl, 1,2,4 triazinyl, 1,2,3 triazinyl and tetrazolyl.

Examples of 6-membered monocyclic heteroaryl groups are pyridinyl, pyridazinyl, pyrimidinyl and pyrazinyl.

In one embodiment, 6-membered monocyclic heteroaryl groups contain 1 or 2 ring members which are =N- atoms (where the remainder of the 6 ring members are carbon atoms).

Bicyclic heteroaromatic groups include fused-ring heteroaromatic groups containing 9-14 ring members wherein 1, 2, 3, 4 or more ring members are independently selected from O, S, N or NR^{N}.

In one embodiment, 9-membered bicyclic heteroaryl groups contain 1 ring member which is an -NR^{N}- group, an -O- atom or an -S- atom and, optionally, 1-3 ring members (*e.g*. 1 or 2 ring members) which are =N- atoms (where the remainder of the 9 ring members are carbon atoms).

Examples of 9-membered fused-ring bicyclic heteroaryl groups are benzofuranyl, benzothiophenyl, indolyl, benzimidazolyl, indazolyl, benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-b]pyridinyl, isoindolyl, indazolyl, purinyl, indolininyl, imidazo[1,2-a]pyridinyl, imidazo[1,5-a]pyridinyl, pyrazolo[1,2-a]pyridinyl, pyrrolo[1,2-b]pyridazinyl and imidazo[1,2-c]pyrimidinyl.

In one embodiment, 10-membered bicyclic heteroaryl groups contain 1-3 ring members which are =N- atoms (where the remainder of the 10 ring members are carbon atoms).

Examples of 10-membered fused-ring bicyclic heteroaryl groups are quinolinyl, isoquinolinyl, cinnolinyl, quinazolinyl, quinoxalinyl, phthalazinyl, 1,6-naphthyridinyl, 1,7-naphthyridinyl, 1,8-naphthyridinyl, 1,5-naphthyridinyl, 2,6-naphthyridinyl, 2,7-naphthyridinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazino[2,3-b]pyrazinyl and pyrimido[4,5-d]pyrimidinyl.

The term "heteroarylalkyl" means alkyl substituted with a heteroaryl group.

Examples of acyl groups include alkyl-C(=O)-, cycloalkyl-C(=O)-, alkenyl-C(=O)-, cycloalkenyl-C(=O)-, heteroalkyl-C(=O)-, heterocycloalkyl-C(=O)-, aryl-C(=O)- or heteroaryl-C(=O)-, in particular, alkyl-C(=O)- and aryl-C(=O)-.

Unless indicated explicitly otherwise, where combinations of groups are referred to herein as one moiety, e.g. arylalkyl, the last mentioned group contains the atom by which the moiety is attached to the rest of the molecule.

Where reference is made to a carbon atom of an alkyl group or other group being replaced by O, S(O)ₜ or N, what is intended is that: 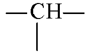 is replaced by 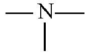
-CH= is replaced by -N=;
=C-H is replaced by =N; or
-CH₂- is replaced by -O-, -S(O)ₜ- or -NR^{N}-.

By way of clarification, in relation to the above mentioned heteroatom containing groups (such as heteroalkyl *etc.*), where a numerical of carbon atoms is given, for instance C₃₋₆heteroalkyl, what is intended is a group based on C₃₋₆alkyl in which one of more of the 3-6 chain carbon atoms is replaced by O, S(O)ₜ or N. Accordingly, a C₃₋₆heteroalkyl group, for example, will contain less than 3-6 chain carbon atoms.

Where mentioned above, R^{N} is H, alkyl, cycloalkyl, aryl, heteroaryl, -C(O)-alkyl, -C(O)-aryl, -C(O)-heteroaryl, -S(O)ₜ-alkyl, -S(O)ₜ-aryl or -S(O)ₜ-heteroaryl. R^{N} may, in particular, be H, alkyl (*e.g.* C₁₋₆alkyl) or cycloalkyl (*e.g.* C₃₋₆cycloalkyl).

Where mentioned above, t is independently 0, 1 or 2, for example 2. Typically, t is 0.

Where a group has at least 2 positions which may be substituted, the group may be substituted by both ends of an alkylene or heteroalkylene chain to form a cyclic moiety.

Optionally substituted groups of the compounds of the invention (*e.g*. alkyl, cycloalkyl, alkoxy, alkenyl, cycloalkenyl, alkynyl, alkylene, alkenylene, heteroalkyl, heterocycloalkyl, heteroalkenyl, heterocycloalkenyl, heteroalkynyl, heteroalkylene, heteroalkenylene, aryl, arylalkyl, arylheteroalkyl, heteroaryl, heteroarylalkyl or heteroarylheteroalkyl groups *etc.*) may be substituted or unsubstituted, in one embodiment unsubstituted. Typically, substitution involves the notional replacement of a hydrogen atom with a substituent group, or two hydrogen atoms in the case of substitution by =O.

Where substituted, there will generally be 1 to 3 substituents, in one embodiment 1 or 2 substituents, in one embodiment 1 substituent.

The optional substituent(s) is/are independently halogen, trihalomethyl, trihaloethyl, - OH, -NH₂, -NO₂, -CN, -N⁺(C₁₋₆alkyl)₂O⁻, -CO₂H, -CO₂C₁₋₆alkyl, -SO₃H, -SOC_{1- 6}alkyl, -SO₂C₁₋₆alkyl, -SO₃C₁₋₆alkyl, -OC(=O)OC₁₋₆alkyl, -C(=O)H, -C(=O)C₁₋₆alkyl, -OC(=O)C_{1- 6}alkyl, =O, -NH(C₁₋₆alkyl), -N(C₁₋₆alkyl)₂, -C(=O)NH₂, -C(=O)N(C_{1- 6}alkyl)₂, -N(C₁₋₆alkyl)C(=O)O(C₁₋₆alkyl), -N(C₁₋₆alkyl)C(=O)N(C₁₋₆alkyl)₂, -OC(=O)N(C_{1- 6}alkyl)₂, -N(C₁₋₆alkyl)C(=O)C₁₋₆alkyl, -C(=S)N(C₁₋₆alkyl)₂, -N(C₁₋₆alkyl)C(=S)C₁₋₆alkyl, -SO₂N(C_{1- 6}alkyl)₂, -N(C₁₋₆alkyl)SO₂C₁₋₆alkyl, -N(C₁₋₆alkyl)C(=S)N(C₁₋₆alkyl)₂, -N(C₁₋₆alkyl)SO₂N(C_{1- 6}alkyl)₂, -C₁₋₆alkyl, -C₁₋₆heteroalkyl, -C₃₋₆cycloalkyl, -C₃₋₆heterocycloalkyl, -C₂₋₆alkenyl, -C₂₋₆heteroalk enyl, -C₃₋₆cycloalkenyl, -C₃₋₆heterocycloalkenyl, -C₂₋₆alkynyl, -C₂₋₆heteroalkynyl, -Z^{u}-C₁₋₆alkyl, - Z^{u}- C₃₋₆cycloalkyl, -Z^{u}-C₂₋₆alkenyl, -Z^{u}-C₃₋₆cycloalkenyl or -Z^{u}-C₂₋₆alkynyl, wherein
Z^{u} is independently O, S, NH or N(C₁₋₆alkyl).

In another embodiment, the optional substituent(s) is/are independently halogen, trihalomethyl, trihaloethyl, -NO₂, -CN, -N⁺(C₁₋₆alkyl)₂O⁻, -CO₂H, -SO₃H, -SOC₁₋₆alkyl, -SO₂C₁₋₆alkyl, -C(=O)H, -C(=O)C₁₋₆alkyl, =O, -N(C₁₋₆alkyl)₂, -C(=O)NH₂, -C₁₋₆alkyl, -C₃₋₆cycloalkyl, -C₃₋₆heterocycloalkyl, -Z^{u}C₁₋₆alkyl or - Z^{u}-C₃₋₆cycloalkyl, wherein Z^{u} is defined above.

In another embodiment, the optional substituent(s) is/are independently halogen, trihalomethyl, -NO₂, -CN, -CO₂H, -C(=O)C₁₋₆alkyl, =O, -N(C₁₋₆alkyl)₂, -C(=O)NH₂, -C₁₋₆alkyl, -C₃₋₆cycloalkyl, -C₃₋₆heterocycloalkyl, -Z^{u}C₁₋₆alkyl or - Z^{u}-C₃₋₆cycloalkyl, wherein Z^{u} is defined above.

In another embodiment, the optional substituent(s) is/are independently halogen, -NO₂, -CN, -CO₂H, =O, -N(C₁₋₆alkyl)₂, -C₁₋₆alkyl, -C₃₋₆cycloalkyl or -C₃₋₆heterocycloalkyl.

In another embodiment, the optional substituent(s) is/are independently halogen, -OH, NH₂, NH(C₁₋₆alkyl), -N(C₁₋₆alkyl)₂, -C₁₋₆alkyl, -C₃₋₆cycloalkyl or -C₃₋₆heterocycloalkyl.

As used herein, the terms "polymers of the invention" and "polymer of formula I" *etc.* include pharmaceutically acceptable derivatives thereof and polymorphs, isomers and isotopically labelled variants thereof.

The term "pharmaceutically acceptable derivative" includes any pharmaceutically acceptable salt, solvate, hydrate or prodrug of a compound of **Formula I**. In one embodiment, the pharmaceutically acceptable derivatives are pharmaceutically acceptable salts, solvates or hydrates of a compound of **Formula I.**

The term "pharmaceutically acceptable salt" includes a salt prepared from pharmaceutically acceptable non-toxic acids or bases including inorganic or organic acids and bases.

Compounds of **Formula I** which contain basic, *e.g.* amino, groups are capable of forming pharmaceutically acceptable salts with acids. In one embodiment, pharmaceutically acceptable acid addition salts of the compounds of **Formula I** include, but are not limited to, those of inorganic acids such as hydrohalic acids (*e.g*. hydrochloric, hydrobromic and hydroiodic acid), sulfuric acid, nitric acid and phosphoric acids. In one embodiment, pharmaceutically acceptable acid addition salts of the compounds of **Formula I** include, but are not limited to, those of organic acids such as aliphatic, aromatic, carboxylic and sulfonic classes of organic acids, examples of which include: aliphatic monocarboxylic acids such as formic acid, acetic acid, propionic acid or butyric acid; aliphatic hydroxy acids such as lactic acid, citric acid, tartaric acid or malic acid; dicarboxylic acids such as maleic acid or succinic acid; aromatic carboxylic acids such as benzoic acid, p-chlorobenzoic acid, phenylacetic acid, diphenylacetic acid or triphenylacetic acid; aromatic hydroxyl acids such as o-hydroxybenzoic acid, p-hydroxybenzoic acid, 1-hydroxynaphthalene-2-carboxylic acid or 3-hydroxynaphthalene-2-carboxylic acid; and sulfonic acids such as methanesulfonic acid, ethanesulfonic acid or benzenesulfonic acid. Other pharmaceutically acceptable acid addition salts of the compounds of **Formula I** include, but are not limited to, those of glycolic acid, glucuronic acid, furoic acid, glutamic acid, anthranilic acid, salicylic acid, mandelic acid, embonic (pamoic) acid, pantothenic acid, stearic acid, sulfanilic acid, algenic acid and galacturonic acid. Wherein the compound of **Formula I** comprises a plurality of basic groups, multiple centres may be protonated to provide multiple salts, e.g. di- or tri-salts of compounds of **Formula** I. For example, a hydrohalic acid salt of a compound of **Formula I** as described herein may be a monohydrohalide, dihydrohalide or trihydrohalide, *etc.* In one embodiment, the salts include, but are not limited to those resulting from addition of any of the acids disclosed above. In one embodiment of the compound of **Formula I**, two basic groups form acid addition salts. In a further embodiment, the two addition salt counterions are the same species, e.g. dihydrochloride, dihydrosulphide etc. Typically, the pharmaceutically acceptable salt is a hydrochloride salt, such as a dihydrochloride salt.

Compounds of **Formula I** which contain acidic, e.g. carboxyl, groups are capable of forming pharmaceutically acceptable salts with bases. In one embodiment, pharmaceutically acceptable basic salts of the compounds of **Formula I** include, but are not limited to, metal salts such as alkali metal or alkaline earth metal salts (*e.g*. sodium, potassium, magnesium or calcium salts) and zinc or aluminium salts. In one embodiment, pharmaceutically acceptable basic salts of the compounds of **Formula I** include, but are not limited to, salts formed with ammonia or pharmaceutically acceptable organic amines or heterocyclic bases such as ethanolamines (*e.g*. diethanolamine), benzylamines, N-methyl-glucamine, amino acids (*e.g*. lysine) or pyridine.

Hemisalts of acids and bases may also be formed, *e.g*. hemisulphate salts.

Pharmaceutically acceptable salts of compounds of **Formula I** may be prepared by methods well-known in the art.

For a review of pharmaceutically acceptable salts, see Stahl and Wermuth, Handbook of Pharmaceutical Salts: Properties, Selection and Use (Wiley-VCH, Weinheim, Germany, 2002).

The compounds of the invention may exist in both unsolvated and solvated forms. The term "solvate" includes molecular complexes comprising a compound of the invention and one or more pharmaceutically acceptable solvent molecules such as water or C₁₋₆ alcohols, *e.g.* ethanol. The term "hydrate" means a "solvate" where the solvent is water.

The compounds of the invention may exist in solid states from amorphous through to crystalline forms. All such solid forms are included within the invention.

Compounds of the invention may exist in one or more geometrical, optical, enantiomeric, diastereomeric and tautomeric forms, including but not limited to *cis-* and *trans*-forms, *E-* and *Z-*forms, *R-, S*- and *meso*-forms, keto- and enol-forms. All such isomeric forms are included within the invention. The isomeric forms may be in isomerically pure or enriched form, as well as in mixtures of isomers (*e.g*. racemic or diastereomeric mixtures).

The invention includes pharmaceutically acceptable isotopically-labelled compounds of **Formula I** wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S. Certain isotopically-labelled compounds of **Formula I,** for example, those incorporating a radioactive isotope, are useful in drug and/or substrate tissue distribution studies. The radioactive isotopes ³H and ¹⁴C are particularly useful for this purpose in view of their ease of incorporation and ready means of detection.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labelled compounds of **Formula I** can generally be prepared by conventional techniques known to those skilled in the art or by processes analogous to those described herein using an appropriate isotopically-labelled reagent in place of the non-labelled reagent previously employed.

It will be appreciated that the polymers, as described herein, may be substituted with any number of substituents or functional moieties. The terms substituted, whether preceded by the term "optionally" or not, and substituent, as used herein, refer to the ability, as appreciated by one skilled in this art, to change one functional group for another functional group provided that the valency of all atoms is maintained. When more than one position in any given structure may be substituted with more than one substituent selected from a specified group, the substituent may be either the same or different at every position. The substituents may also be further substituted (e.g., an aryl group substituent may have another substituent off it, such as another aryl group, which is further substituted with fluorine at one or more positions).

The term thiohydroxyl or thiol, as used herein, refers to a group of the formula -SH.

The present invention further provides a composition comprising an active agent and a polymer of formula I. The composition may comprise nanoparticles and/or microparticles containing the active agent and the polymer. The composition may comprise two or more different polymers as defined in formula I. For example, the composition may comprise polymers of formula I wherein R₁ and R₂ are both CysLysLysLys and polymers of formula I wherein R₁ and R₂ are both CysHisHisHis.

The active agent may be a polynucleotide, protein or small molecule. Typically, the active agent is a polynucleotide. The polynucleotide may be selected from the group consisting of DNA, RNA, siRNA and miRNA, preferably from the group consisting of siRNA and miRNA. In an embodiment, the polynucleotide is selected from the group consisting of DNA, RNA and siRNA.

Typically, a polynucleotide comprises at least three nucleotides. Preferably, the polynucleotide is 20-30 nucleotides in length, more preferably 20-25 nucleotides in length, for example, 22 nucleotides in length.

The polynucleotide may be derived from natural nucleosides (i.e., adenosine, thymidine, guanosine, cytidine, uridine, inosine, xanthosine, deoxyadenosine, deoxythymidine, deoxyguanosine, deoxyinosine, and deoxycytidine), nucleoside analogues (e.g., 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2- thiocytidine), or mixtures thereof. The nucleotides may be derived from chemically modified bases, biologically modified bases (e.g., methylated bases), intercalated bases, unmodified or modified sugars (e.g., 2 '-fluororibose, ribose, 2'-deoxyribose, arabinose, and hexose), and/or unmodified or modified phosphate groups (e.g., phosphorothioates and 5' -N-phosphoramidite linkages).

As used herein, the term "small molecule" refers to organic compounds, whether naturally-occurring or artificially created (e.g., via chemical synthesis) that have relatively low molecular weight and that are not proteins, polypeptides, or polynucleotides. Typically, small molecules have a molecular weight of less than about 1500 g/mol.

The present invention provides nanoparticles according to claim 14. Further discussion of the nanoparticles of the present invention follows. It will be understood that the discussion applies equally to microparticles.

The nanoparticles may comprise a polynucleotide and polymers of formula I wherein the or each oligopeptide has a net positive charge at pH 7. The positively charged oligopeptides interact with negatively charged polynucleotide during the process of nanoparticle formation and facilitate encapsulation of the polynucleotide in the nanoparticles.

The nanoparticles may comprise polymers of formula I wherein the or each oligopeptide has a net negative charge at pH 7 and an active agent that has a net positive charge at pH7. The negatively charged oligopeptides interact with positively charged active agent during the process of nanoparticle formation and facilitate encapsulation of the active agent in the nanoparticles.

The nanoparticles comprise a mixture of different polymers of the invention. The nanoparticles comprise
(a) a polymer according to formula I wherein the or each oligopeptide has a net positive charge at pH 7; and
(b) a polymer according to formula I wherein the or each oligopeptide has a net negative charge at pH 7.

Thus, the invention provides nanoparticles with net surface charge that may be varied by modifying the proportions of polymers (a) and (b) above. The ratio of (a) to (b) may be 1:99, 5:95, 10:90, 25:75, 50:50, 75:25, 90:10, 95:5, or 99:1 by weight.

Such nanoparticles are suitable for both drug and polynucleotide encapsulation and show improved pharmacological properties.

The inclusion of a population of polymers modified with oligopeptides that have a net negative charge at pH 7 facilitates encapsulation by nanoprecipitation of shorter DNA and RNA sequences. Shorter DNA and RNA sequences show lower encapsulation efficiency and/or lower absolute loading than longer sequences when used in a nanoprecipitation step with PBAEs known in the art. The inventors have found that the addition of other polyanionic species, such as the negatively charged polymers described here, helps in the assembly during the nanoprecipitation process of the resulting nanoparticles containing polymer and polynucleotide.

This is especially useful for the encapsulation of short polynucleotides, such as siRNA and miRNA sequences, which have a length of approximately 20 to 30 base pairs and are unstable during circulation in the body. The incorporation of short polynucleotides such as siRNA and miRNA into nanoparticles has previously presented difficulties owing to their lower charge.

The inventors have found that the use of polymers according to formula I or formula II wherein the or each oligopeptide has a net positive charge at pH 7 in combination with polymers according to formula I or formula II wherein the or each oligopeptide has a net negative charge at pH 7 allows the loading of short polynucleotides such as siRNA or miRNA into nanoparticles with high encapsulation efficiency and high loading. Further, the use of the two types of polymers described above prevents degradation of the short polynucleotides and allows more efficient transfection. It is thought that the positively charged oligonucleotides "wrap" around the negatively charged polynucleotides, and the negatively charged oligonucleotides "wrap" around the positively charged oligonucleotides to neutralize the excess charge (referred to by the inventors as the "mantle effect").

Further, the inclusion of polymers modified with oligopeptides that have a net negative charge at pH 7 facilitates delivery of the nanoparticles through complex body barriers, such as intestinal and pulmonary mucosa, as the net surface charge changes may vary during the interaction with those barriers.

Nanoparticles of the present invention may be formed with high active agent content and high encapsulation efficiency.

Herein, the active agent encapsulation efficiency refers to the active agent incorporated into the nanoparticles as a weight percentage of the total active agent used in the method of preparation of the active agent-containing nanoparticles. It is typically up to and including 95%, more typically from 70% to 95%.

Herein, active agent entrapment refers to the weight percentage of the active agent in the active agent-loaded nanoparticles. Active agent entrapment is preferably at least 2 wt%, more preferably at least 5 wt%, more preferably at least 10 wt% and typically in the range of from 2 wt% to 20 wt%, more preferably from 5 wt% to 20 wt%, more preferably from 10 wt% to 20 wt%.

When the composition comprises nanoparticles, preferably, the nanoparticles constitute from about 1% to about 90% by weight of the composition. More preferably, the nanoparticles constitute about 5% to about 50% by weight of the composition, more preferably, about 10% to about 30%. The composition may further comprise a vehicle. The vehicle may be any pharmaceutically acceptable diluent or excipient, as known in the art. The vehicle is typically pharmacologically inactive. Preferably, the vehicle is a polar liquid. Particularly preferred vehicles include water and physiologically acceptable aqueous solutions containing salts and/or buffers, for example, saline or phosphate-buffered saline. Optionally, the vehicle is a biological fluid. A liquid vehicle may be removed by, for example, lyophilization, evaporation or centrifugation for storage or to provide a powder for pulmonary or nasal administration, a powder for suspension for infusion, or tablets or capsules for oral administration.

The active agent(s) may be present within the nanoparticles or on the surfaces of the nanoparticles. Typically the nanoparticles are present within the nanoparticles. The interaction between the active agent(s) and the nanoparticle is typically non-covalent, for example, hydrogen bonding, electrostatic interaction or physical encapsulation. Typically the interaction is electrostatic

The nanoparticles are biocompatible and sufficiently resistant to their environment of use that a sufficient amount of the nanoparticles remain substantially intact after entry into the mammalian body so as to be able to reach the desired target and achieve the desired physiological effect. The polymers described herein are biocompatible and preferably biodegradable.

Herein, the term 'biocompatible' describes as substance which may be inserted or injected into a living subject without causing an adverse response. For example, it does not cause inflammation or acute rejection by the immune system that cannot be adequately controlled. It will be recognized that "biocompatible" is a relative term, and some degree of immune response is to be expected even for substances that are highly compatible with living tissue. An *in vitro* test to assess the biocompatibility of a substance is to expose it to cells; biocompatible substances will typically not result in significant cell death (for example, >20%) at moderate concentrations (for example, 29 µg/10⁴ cells).

Herein, the term 'biodegradable' describes a polymer which degrades in a physiological environment to form monomers and/or other non-polymeric moieties that can be reused by cells or disposed of without significant toxic effect. Degradation may be biological, for example, by enzymatic activity or cellular machinery, or may be chemical, typically a chemical process that takes place under physiological conditions. Degradation of a polymer may occur at varying rates, with a half-life in the order of days, weeks, months, or years, depending on the polymer or copolymer used. The components preferably do not induce inflammation or other adverse effects *in vivo.* In certain preferred embodiments, the chemical reactions relied upon to break down the biodegradable compounds are uncatalysed.

Herein, the term 'nanoparticles' refers to a solid particle with a diameter of from about 1 to about 1000nm. Herein, the term 'microparticles' refers to a solid particle with a diameter of from about 1µm to about 100µm. The mean diameter of the nanoparticles of the present invention may be determined by methods known in the art, preferably by dynamic light scattering. In particular, the invention relates to nanoparticles that are solid particles with a diameter of from about 1 to about 1000nm when analysed by dynamic light scattering at a scattering angle of 90° and at a temperature of 25°C, using a sample appropriately diluted with filtered water and a suitable instrument such as the Zetasizer™ instruments from Malvern Instruments (UK) according to the standard test method ISO 22412:2008 (cumulants method A.1.3.2). Where a particle is said to have a diameter of *x* nm, there will generally be a distribution of particles about this mean, but at least 50% by number (e.g. >60%, >70%, >80%, >90%, or more) of the particles will have a diameter within the range *x*±20%.

Preferably, the diameter of the nanoparticle is from about 10 to about 1000nm, more preferably from about 5 to about 500nm, more preferably from about 50 to about 400nm, more preferably from about 50 to about 150nm. Alternatively, the diameter of the nanoparticle is from about 1 to about 100nm. In one embodiment, the nanoparticles exhibit a degree of agglomeration of less than 10%, preferably less than 5 %, preferably less than 1%, and preferably the nanoparticles are substantially non-agglomerated, as determined by transmission electron microscopy.

The present invention further provides a method of encapsulating an agent in a matrix of polymer of formula I or formula II to form nanoparticles, the method comprising steps of: providing an agent; providing the polymer; and contacting the agent and the polymer under suitable conditions to form nanoparticles. In particular, the agent and polymer may be mixed in solution at concentrations appropriate to obtain the desired ratio, mixed vigorously and then incubated in an oven at about 37ºC for about 30 minutes.

The present invention further provides a method of synthesizing a polymer of formula I comprising the steps of reacting a compound of **Formula II** wherein L₃ is as defined above, with a primary amine of formula L₄H₂, wherein L₄ is as defined above, to produce a polymer of Formula II as shown in Scheme 2.

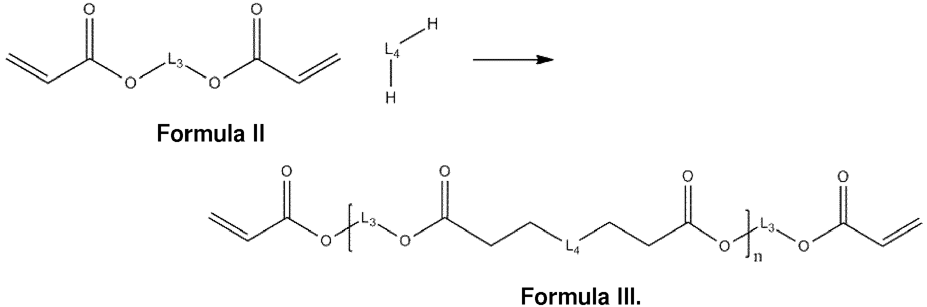

The compound of **Formula III** is further reacted with compounds of **Formula IV** to form a compound of Formula V: 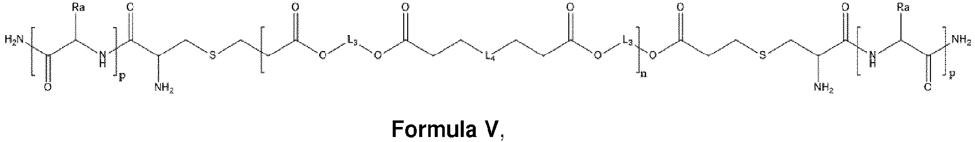 wherein p and Rₐ independently at each occurrence are selected from the lists defined above. In some cases, each occurrence of p is the same and the Rₐ groups are selected such that the sequence of Rₐ groups starting from the sulfur linkage is the same at each end of the compound, that is, p and Rₐ are selected such that the polymer has two-fold symmetry about L₄.

In an alternative to the above step, the compound of **Formula III** is further reacted with compounds of formula H₂NR_{y}, wherein R_{y} is as defined above, and compounds of Formula IV and the resulting mixture is separated to obtain a compound of Formula VI: 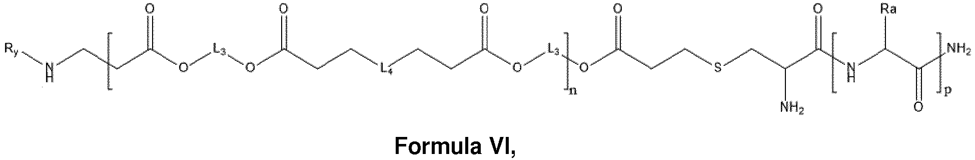 wherein Rₐ is independently selected at each occurrence from the lists defined above and p is as defined above.

It will be recognized that further methods of attaching an oligopeptide to the compound of **Formula III** would be available to the skilled person, who would be aware of appropriate nucleophiles for reaction at the terminal acrylate groups of **Formula III.**

### BRIEF DESCRIPTION OF THE FIGURES

Figure 1 shows the hydrodynamic diameter and zeta potential of polymer-DNA complexes prepared using different oligopeptide end-modified PBAEs and pGFP plasmid at 50:1 ratio (w/w).
Figure 2 shows agarose gel electrophoresis analysis of oligopeptide end-modified PBAEs and reference polymers complexed with DNA.
Figure 3 shows the buffering capacity of oligopeptide end-modified PBAEs and reference polymers.
Figure 4 shows flow cytometry analysis of GFP expression in (a) cos-7, (b) hnDf and (c) HaCaT cells transfected with different oligopeptide end-modified PBAEs and a reference polymer.
Figure 5 shows the viability of cos-7 cells transfected with different oligopeptide end-modified PBAEs.
Figure 6 shows the hydrodynamic diameter and zeta potential of polymer-siRNA complexes prepared using different oligopeptide end-modified PBAEs and GFP-specific siRNA at 200:1 ratio (w/w).
Figure 7 shows flow cytometry analysis of GFP expression silencing in MDA-MB-231/GFP cells transfected with GFP-specific siRNA using different oligopeptide end-modified PBAEs.
Figure 8 shows the degree of encapsulation of bovine insulin using glutamic acid and lysine end-modified PBAEs at a final polymer:protein ratio of 200:1 (w/w).

The invention is further illustrated by the following examples. It will be appreciated that the examples are for illustrative purposes only and are not intended to limit the invention as described above. Modification of detail may be made without departing from the scope of the invention.

### EXAMPLES

### Materials

Reagents and solvents were obtained from Sigma-Aldrich and Panreac and used as received unless otherwise stated. Plasmid pmaxGFP (3486 bp) was obtained from Amaxa. Cell lines were obtained from ATCC (Manassas, VA) and maintained at 37°C in 5% CO2 atmosphere in complete DMEM, containing 10% fetal bovine serum, 100 units/ml penicillin, 100 ug/mL streptomycin, 0.1 mM MEM Non-Essential Amino Acids (NEAA), 2 mM L-glutamine obtained from Gibco.

### Example 1: Synthesis of PBAE polymers

Poly(*β*-aminoester)s were synthesized following a two-step procedure, described in the literature (e.g. in Montserrat, N. et al. J. Biol. Chem. 286, 12417-12428 (2011)). First, an acrylate-terminated polymer was synthesized by addition reaction of primary amines with diacrylates (at 1:1.2 molar ratio of amine:diacrylate). Finally, PBAEs were obtained by end-capping modification of the resulting acrylate-terminated polymer with different kind of amine- and thiol-bearing moieties. Synthesized structures were confirmed by ¹H-NMR and FT-IR analysis. NMR spectra were recorded in a 400 MHz Varian (Varian NMR Instruments, Claredon Hills, IL) and methanol-d₄ was used as solvent. IR spectra were obtained using a Nicolet Magna 560 (Thermo Fisher Scientific, Waltham, MA) with a KBr beamsplitter, using methanol as solvent in evaporated film. Molecular weight determination was conducted on a Hewlett-Packard 1050 Series HPLC system equipped with two GPC Ultrastyragel columns, 10³ and 10⁴ Å (5 µm mixed, 300 mm x 19 mm, Waters Millipore Corporation, Milford, MA, USA) and THF as mobile phase. The molecular weight was calculated by comparison with the retention times of polystyrene standards.

### Example 2: Synthesis of acrylate terminated intermediate

1,4-butanediol diacrylate (8.96g, 4.07×10⁻² mol) and 5-amino-1-pentanol (3.5g, 3.39×10⁻² mol) were mixed in a vial. The mixture was stirred at 90°C for 24h, and then cooled to room temperature to form a slightly yellow viscous solid, the acrylate terminated intermediate (designated C32). Intermediate C32 was stored at 4°C before being used in subsequent steps.

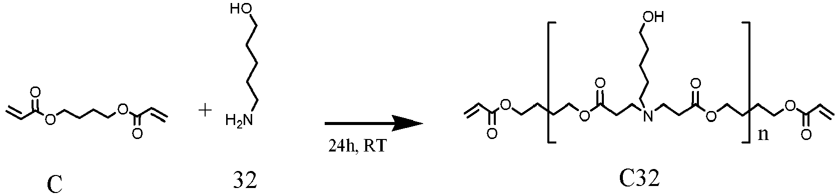

### Example 3 (comparative): Synthesis of PBAEs end-modified with primary amines

PBAEs end-modified with primary amines were prepared as defined in Zugates, G.T. et al. Bioconjugate Chem. 18, 1887-1896 (2007). A solution of intermediate C32 (1 g, 0.5 mmol) in THF (2 ml) was mixed with a solution of 1,5-diamino-2-methyl-pentane (0.24 g, 0.271 ml, 2 mmol) in THF (8 ml). The mixture was stirred overnight at room temperature, then was precipitated in diethyl ether (100 ml) and finally was dried in vacuum.

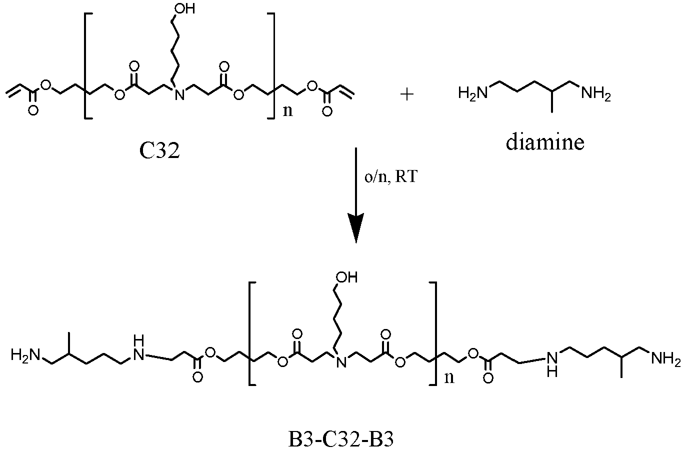

### Example 4 (comparative): Further synthesis of PBAEs end-modified with primary amines

Diamine end-modified poly(β-aminoester), B3, was synthesized following a procedure described elsewhere (Zugates, G.T. et al. Bioconj. Chem. 18 1887-1896 (2007), Yang, F. et al., Proc. Natl Acad. Sci. USA. 107 3317-3322 (2010), Sunshine, J.C. Biomacromolecules 12 3592-3600 (2011)). Briefly, 5-amino-1-pentanol (3.44 g, 33 mmol) and 1,4-butanediol diacrylate (7.93 g, 40 mmol) were polymerized under magnetic stirring at 90°C for 24 hours. The resulting acrylate-terminated polymer C32 (1 g, 0.4 mmol) and 2-methyl-1,5-pentanediamine (0.23 g, 0.27 mL, 2 mmol) were dissolved in tetrahydrofuran and stirred overnight at room temperatures. The resulting diamine end-modified polymer B3 was isolated by precipitation in diethyl ether and dried under vacuum.
**IR** (evaporated film): v = 1055, 1089, 1125, 1196 (C-O), 1257, 1463, 1733 (C=O), 2079, 2191, 2253, 2861, 2936, 3398 (N-H, O-H) cm⁻¹
**¹H-NMR** (400 MHz, CD₃OD, TMS) (ppm): δ = 4.11 (t, CH₂-CH₂-O), 3.72 (t), 3,55 (t, CH₂-CH₂-OH), 2.87 (t, -NH-CH₂-CH₂-C(=O)-), 2.77 (t, CH₂-CH₂-N-), 2.60-2.51 (br, -NH-CH₂-(CH₂)₂-CH(CH₃)-NH-), 2.46 (br, >N-CH₂-(CH₂)₄-OH, >N-CH₂-CH₂-C(=O)-O), 1.87 (br), 1.73 (br), 1.60-1.41 (br, -O-CH₂-CH₂-CH₂-CH₂-O, -CH₂-CH₂-OH, -CH₂-CH₂-NH₂), 1.35 (br, -N-CH₂-CH₂-CH₂-(CH₂)₂-OH), 0.94 (d, CH₃-CH< from diamine).

### Example 5: Synthesis of PBAEs end modified with oligopeptides

In general, oligopeptide-modified PBAEs were obtained as follows: acrylate-terminated polymer C32 or C32SS and either amine- or thiol-terminated oligopeptide (for example, HS-Cys-Arg-Arg-Arg (CR3), H₂N-Arg-Arg-Arg (R3) or HS-Cys-Glu-Glu-Glu (CE3) - other oligopeptides are indicated by similar abbreviations using the standard one-letter code) were mixed at 1:2 molar ratio in DMSO. The mixture was stirred overnight at room temperature and the resulting polymer was obtained by precipitation in diethyl ether:acetone (3:1).
(a) The following synthetic procedure to obtain tri-arginine end-modified PBAEs is shown as an example: Intermediate C32 was prepared as described in Example 1 above. A solution of intermediate C32 (0.15 g, 0.075 mmol) in DMSO (2 ml) was mixed with the corresponding solution of oligopeptide (Cys-Arg-Arg-Arg (CR3; 0.11 g, 0.15 mmol) in DMSO (1 mL) in an appropriate molar ratio, 1:2 respectively. The mixture was stirred overnight at room temperature, then was precipitated in diethyl ether/acetone (3:1). 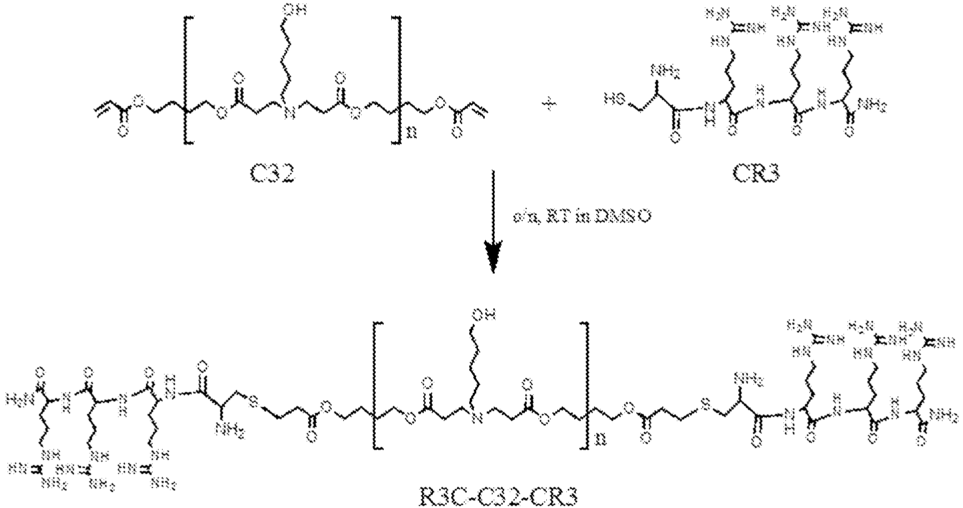 **IR** (evaporated film): v = 721, 801, 834, 951, 1029, 1133 (C-O), 1201, 1421, 1466, 1542, 1672 (C=O, from peptide amide), 1731 (C=O, from ester), 2858, 2941, 3182, 3343 (N-H, O-H) cm⁻¹ **¹H-NMR** (400 MHz, CD₃OD, TMS) (ppm): δ = 4.41-4.33 (br, NH₂-C(=O)-CH-NH-C(=O)-CH-NH-C(=O)-CH-NH-C(=O)-CH-CH₂-, 4.11 (t, CH₂-CH₂-O), 3.55 (t, CH₂-CH₂-OH), 3.22 (br, NH₂-C(=NH)-NH-CH₂-, OH-(CH₂)₄-CH₂-N-), 3.04 (t, CH₂-CH₂-N-), 2.82 (dd, -CH₂-S-CH₂), 2.48 (br, -N-CH₂-CH₂-C(=O)-O), 1.90 (m, NH₂-C(=NH)-NH-(CH₂)₂-CH₂-CH-), 1.73 (br, -O-CH₂-CH2-CH2-CH₂-O), 1.69 (m, NH₂-C(=NH)-NH-CH₂-CH₂-CH₂-), 1.56 (br, -CH₂-CH₂-CH₂-CH₂-OH), 1.39 (br, -N-(CH₂)₂-CH₂-(CH₂)₂-OH).
(b) Tri-lysine modified oligopeptides (K3C-C32-CK3) were prepared according to the same protocol and characterized as follows:
   **IR** (evaporated film): v = 721, 799, 834, 1040, 1132, 1179 (C-O), 1201, 1397, 1459, 1541, 1675 (C=O, from peptide amide), 1732 (C=O, from ester), 2861, 2940, 3348 (N-H, O-H) cm⁻¹ **¹H-NMR** (400 MHz, CD₃OD, TMS) (ppm): δ = 4.38-4.29 (br, NH₂-(CH₂) ₄-CH-), 4.13 (t, CH₂-CH₂-O-),3.73 (br,NH₂-CH-CH₂-S-), 3.55 (t, CH₂-CH₂-OH), 2.94 (br, CH₂-CH₂-N-, NH₂-CH₂-(CH₂)₃-CH-), 2.81 (dd, -CH₂-S-CH₂), 2.57 (br, -N-CH₂-CH₂-C(=O)-O), 1.85 (m, NH₂-(CH₂)₃-CH₂-CH-), 1.74 (br, - O-CH₂-CH2-CH2-CH₂-O), 1.68 (m, NH₂-CH₂-CH₂-(CH₂)₂-CH-), 1.54 (br, -CH₂-CH₂-CH₂-CH₂-OH), 1.37 (br, -N-(CH₂)₂-CH₂-(CH₂)₂-OH).
(c) Tri-histidine modified oligopeptides (H3C-C32-CH3) were prepared according to the same protocol and characterized as follows: **IR** (evaporated film): v = 720, 799, 832, 1040, 1132, 1201, 1335, 1403, 1467, 1539, 1674 (C=O, from peptide amide), 1731 (C=O, from ester), 2865, 2941, 3336 (N-H, O-H) cm⁻¹
   **¹H-NMR** (400 MHz, CD₃OD, TMS) (ppm): δ = 8.0-7.0 (br -N(=CH)-NH-C(=CH)-) 4.61-4.36 (br, - CH2-CH-), 4.16 (t, CH₂-CH₂-O-), 3.55 (t, CH₂-CH₂-OH), 3.18 (t, CH₂-CH₂-N-, 3.06 (dd, -CH₂-CH-), 2.88 (br, OH-(CH₂)₄-CH₂-N-), 2.82 (dd, -CH₂-S-CH₂-), 2.72 (br, -N-CH₂-CH₂-C(=O)-O), 1.75 (br, -O-CH₂-CH2-CH2-CH₂-O), 1.65 (m, NH₂-CH₂-CH₂-(CH₂)₂ -CH-), 1.58 (br, -CH₂-CH₂-CH₂-CH₂-OH), 1.40 (br, -N-(CH₂)₂-CH₂-(CH₂)₂-OH).

### Example 6: Synthesis of PBAEs with asymmetric end modifications

In general, asymmetric oligopeptide-modified PBAEs were obtained as follows: Acrylate-terminated polymer C32 (or C32SS) and either amine- or thiol-terminated oligopeptide (for example, CR3, R3 or CE3) were mixed at 1:1 molar ratio in DMSO. The mixture was stirred overnight at room temperature. Equimolar amount of a second amine- or thiol-terminated oligopeptide, or of a primary amine, was added and the mixture was stirred overnight at room temperature. The resulting asymmetric PBAE polymers were obtained by precipitation in diethyl ether/acetone (3:1). The following synthetic procedure to obtain asymmetric end-modified B3-C32-CR3 PBAEs is shown as an example: a solution of intermediate C32 (0.15 g, 0.075 mmol) in DMSO (2 mL) was mixed with the corresponding solution of oligopeptide Cys-Arg-Arg-Arg (CR3; 0.055 g, 0.075 mmol) in DMSO (1 ml) and was stirred overnight at room temperature. Subsequently, 2-methyl-1,5-pentanediamine (0.017 g, 0.02 ml, 0.15 mmol) was added in the mixture for 4h at room temperature in DMSO. A mixture of asymmetric end-modified polymer B3-C32-CR3 with B3-C32-B3 and R3C-C32-CR3 was obtained by precipitation overnight in diethyl ether/acetone (3:1). The mixture may be used without further purification or the asymmetric end-modified polymer B3-C32-CR3 may be separated from the mixture by standard methods.

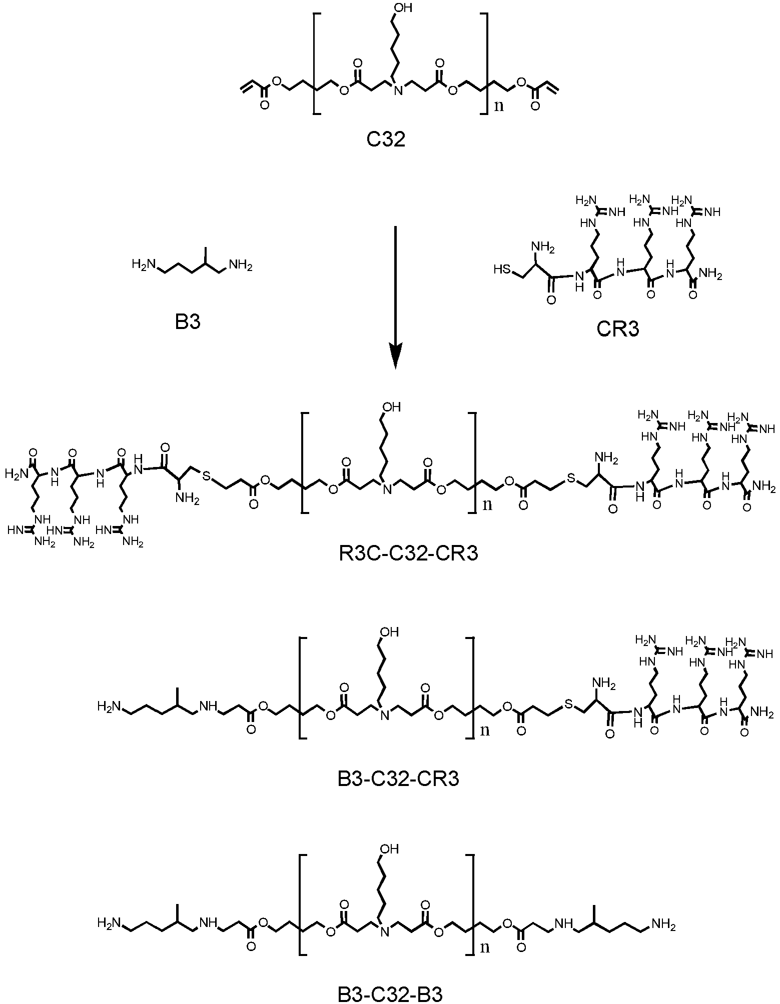

### Example 1: Library of compounds

A library of different oligopeptide end-modified PBAEs was synthesized by adding primary amines to diacrylates followed by end-modification. According to Formula I, the oligopeptide end-modified PBAEs shown in Table 1 were synthesized.

**Table 1: Library of oligopeptide end-modified PBAEs**

| Polymer | L₃ | L₄ | HL₁-R₁ | HL₂-R₂ |
|---|---|---|---|---|
| B3 (reference) | -CH₂-(CH₂)₂-CH₂- | >N-(CH₂)₅-OH | NH₂-CH₂-(CH₂)₂-CH(CH₃)-CH₂-NH₂ | H₂N-CH₂-(CH₂)₂-CH(CH₃)-CH₂-NH₂ |
| R3-C32-R3 | -CH₂-(CH₂)₂-CH₂- | >N-(CH₂)₅-OH | H₂N-Arg-Arg-Arg | H₂N-Arg-Arg-Arg |
| K3-C32-K3 | -CH₂-(CH₂)₂-CH₂- | >N-(CH₂)₅-OH | NH₂-Lys-Lys-Lys | H_{2N}-Lys-Lys-Lys |
| H3-C32-H3 | -CH₂-(CH₂)₂-CH₂- | >N-(CH₂)₅-OH | NH₂-His-His-His | NH2-His-His-His |
| R3C-C32-CR3 | -CH₂-(CH₂)₂-CH₂- | >N-(CH₂)₅-OH | HS-Cys-Arg-Arg-Arg | HS-Cys-Arg-Arg-Arg |
| K3C-C32-CK3 | -CH₂-(CH₂)₂-CH₂- | >N-(CH₂)₅-OH | HS-Cys-Lys-Lys-Lys | HS-Cys-Lys-Lys-Lys |
| H3C-C32-CH3 | -CH₂-(CH₂)₂-CH₂- | >N-(CH₂)₅-OH | HS-Cys-His-His-His | HS-Cys-His-His-His |
| B3-C32-R3 | -CH₂-(CH₂)₂-CH₂- | >N-(CH₂)₅-OH | H₂N-CH₂-(CH₂)₂-CH(CH₃)-CH₂-NH₂ | H₂N-Arg-Arg-Arg |
| B3-C32-CR3 | -CH₂-(CH₂)₂-CH₂- | >N-(CH₂)₅-OH | H₂N-CH₂-(CH₂)₂-CH(CH₃)-CH₂-NH₂ | HS-Cys-Arg-Arg-Arg |
| B3-C32-CK3 | -CH₂-(CH₂)₂-CH₂- | >N-(CH₂)₅-OH | H₂N-CH₂-(CH₂)₂-CH(CH₃)-CH₂-NH₂ | HS-Cys-Lys-Lys-Lys |
| B3-C32-CH3 | -CH₂-(CH₂)₂-CH₂- | >N-(CH₂)₅-OH | H₂N-CH₂-(CH₂)₂-CH(CH₃)-CH₂-NH₂ | HS-Cys-His-His-His |
| R3C-C32-CK3 | -CH₂-(CH₂)₂-CH₂- | >N-(CH₂)₅-OH | HS-Cys-Arg-Arg-Arg | HS-Cys-Lys-Lys-Lys |
| R3C-C32-CH3 | -CH₂-(CH₂)₂-CH₂- | >N-(CH₂)₅-OH | HS-Cys-Arg-Arg-Arg | HS-Cys-His-His-His |
| K3C-C32-CH3 | -CH₂-(CH₂)₂-CH₂- | >N-(CH₂)₅-OH | HS-Cys-Lys-Lys-Lys | HS-Cys-His-His-His |
| R3C-C32SS-CR3 | -CH₂-CH₂-S-S-CH₂-CH₂- | >N-(CH₂)₅-OH | HS-Cys-Arg-Arg-Arg | HS-Cys-Arg-Arg-Arg |
| K3C-C32SS-CK3 | -CH₂-CH₂-S-S-CH₂-CH₂- | >N-(CH₂)₅-OH | HS-Cys-Lys-Lys-Lys | HS-Cys-Lys-Lys-Lys |
| H3C-C32SS-CH3 | -CH₂-CH₂-S-S-CH₂-CH₂- | >N-(CH₂)₅-OH | HS-Cys-His-His-His | HS-Cys-His-His-His |
| B3-C32SS-CR3 | -CH₂-CH₂-S-S-CH₂-CH₂- | >N-(CH₂)₅-OH | H₂N-CH₂-(CH₂)₂-CH(CH₃)-CH₂-NH₂ | HS-Cys-Arg-Arg-Arg |
| B3-C32SS-CK3 | -CH₂-CH₂-S-S-CH₂-CH₂- | >N-(CH₂)₅-OH | H₂N-CH₂-(CH₂)₂-CH(CH₃)-CH₂-NH₂ | HS-Cys-Lys-Lys-Lys |
| B3-C32SS-CH3 | -CH₂-CH₂-S-S-CH₂-CH₂- | >N-(CH₂)₅-OH | H₂N-CH₂-(CH₂)₂-CH(CH₃)-CH₂-NH₂ | HS-Cys-His-His-His |
| R3C-C32SS-CK3 | -CH₂-CH₂-S-S-CH₂-CH₂- | >N-(CH₂)₅-OH | HS-Cys-Arg-Arg-Arg | HS-Cys-Lys-Lys-Lys |
| R3C-C32SS-CH3 | -CH₂-CH₂-S-S-CH₂-CH₂- | >N-(CH₂)₅-OH | HS-Cys-Arg-Arg-Arg | HS-Cys-His-His-His |
| K3C-C32SS-CH3 | -CH₂-CH₂-S-S-CH₂-CH₂- | >N-(CH₂)₅-OH | HS-Cys-Lys-Lys-Lys | HS-Cys-His-His-His |
| D3C-C32-CD3 | -CH₂-(CH₂)₂-CH₂- | >N-(CH₂)₅-OH | HS-Cys-Asp-Asp-Asp | HS-Cys-Asp-Asp-Asp |
| E3C-C32-CE3 | -CH₂-(CH₂)₂-CH₂- | >N-(CH₂)₅-OH | HS-Cys-Glu-Glu-Glu | HS-Cys-Glu-Glu-Glu |
| D3C-C32-CE3 | -CH₂-(CH₂)₂-CH₂- | >N-(CH₂)₅-OH | HS-Cys-Asp-Asp-Asp | HS-Cys-Glu-Glu-Glu |
| E3C-C32SS-CD3 | -CH₂-CH₂-S-S-CH₂-CH₂- | >N-(CH₂)₅-OH | HS-Cys-Asp-Asp-Asp | HS-Cys-Asp-Asp-Asp |
| E3C-C32SS-CE3 | -CH₂-CH₂-S-S-CH₂-CH₂- | >N-(CH₂)₅-OH | HS-Cys-Glu-Glu-Glu | HS-Cys-Glu-Glu-Glu |
| D3C-C32SS-CE3 | -CH₂-CH₂-S-S-CH₂-CH₂- | >N-(CH₂)₅-OH | HS-Cys-Asp-Asp-Asp | HS-Cys-Glu-Glu-Glu |

### Example 8: Formation and characterization of polymer-DNA complexes

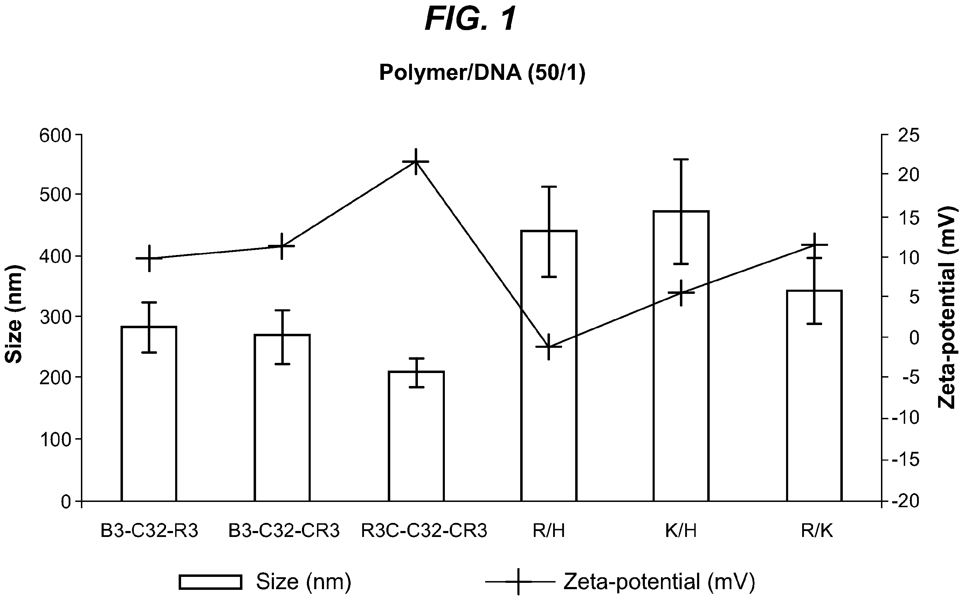

Stock solutions of all polymers were prepared in DMSO (100mg/ml). These polymer solutions were diluted (25mM acetate buffer pH 5.0) at appropriate concentration to obtain the desired ratio polymer-DNA (w/w). Then 100µl of diluted polymer was added to 100µl of plasmid DNA (60µg/mL in acetate buffer 25mM pH 5.0), mixed with vortex vigorously for few seconds then incubated in oven at 37°C for 30min. The resulting nanoparticles were diluted in phosphate buffered saline for nanoparticle characterization. Polymer-DNA complexes were characterized in terms of size and zeta potential using dynamic light scattering (Zetasizer nano zs90, Malvern Instruments). The results are shown in figure 1.

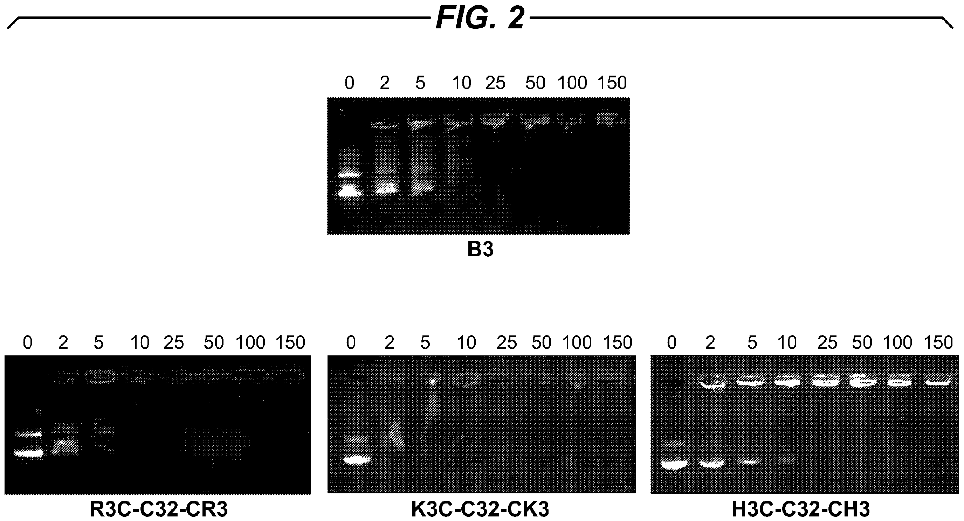

The nanoparticles were also characterized by agarose gel electrophoresis. To assess plasmid retardation, PBAE-DNA complexes containing 0.48 µg of pGFP at different w/w ratios were added to wells of agarose gel (0.8%, containing 1 µg/mL ethidium bromide). Samples were run at 60 V for 45 min (Apelex PS 305, France) to resolve plasmid retardation and visualized by UV illumination. The results are shown in figure 2.

### Example 9: proton sponge effect

The proton sponge effect is a phenomenon that has been shown to facilitate endosomal escape and is mediated by polymers with high buffering capacity, resulting in increased transfection efficiency (Varkouhi, A.K. et al, J. Control. Rel. 151 220-228 (2011).). In general, polymers having tertiary amines in their structure show a buffering effect at the endosomal pH range between 5.0 and 7.5, which causes an increase in osmotic pressure that results in disruption of the endosome Behr, J. Chimia 2 34-36 (1997)). According to the proton sponge effect, the buffering capacity of the newly synthesized poly(β-amino ester)s was determined by acid titration of polymer solutions (figure 2).

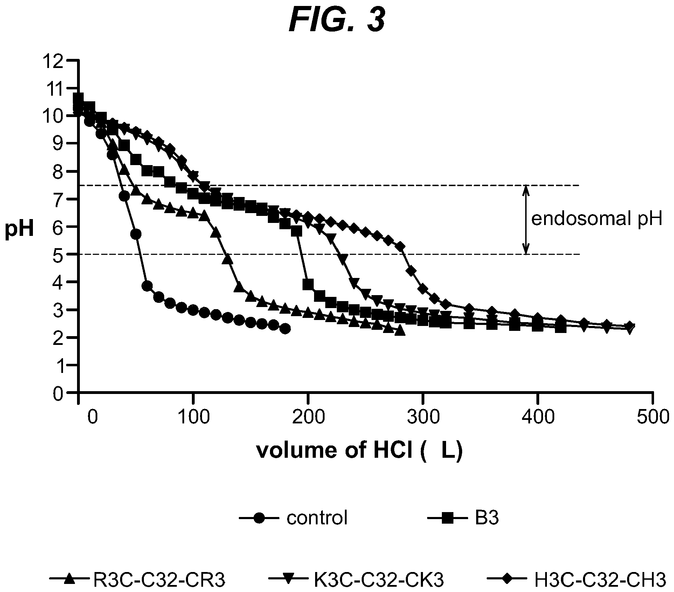

The buffer capacity of polymers was determined by acid-base titration. Briefly, polymers were dissolved at a final concentration of 1mg/mL in an aqueous solution of sodium chloride (150 mM). The resulting polymer solution was adjusted to pH 10 with sodium hydroxide. The titration curve was determined by stepwise addition of 10 µL aliquots of hydrochloric acid (0.1 M). The pH was measured after each addition with a pH meter (Crison Basic 20+, Crison Instruments) until pH 2 was reached. A solution that does not contain polymer was titrated as a control. The results are shown in figure 3.

First, the buffering effect of poly(β-amino ester) B3 was determined, showing suitable buffering capacity down to pH 5.8. The highest buffering capacity was observed with histidine-terminated poly(β-amino ester), which demonstrated high buffering in the pH range between 7.5 and 5.3. Lysine-modified poly(β-amino ester)s presented suitable buffering capacity until pH 5.9. In contrast, poly(β-amino ester)s end-capped with arginine oligopeptides only showed limited buffering capacity in the range between 7.4 and 6.4. Since all polymers stem from the same acrylate-terminated pre-polymer C32, the additional buffering capacity observed results from the amine-rich terminations.

### Example 10: transfection efficacy

The transfection efficacy of the polymers of the present invention and known polymers was compared by assessing the efficiency of delivery of a plasmid encoding green fluorescent protein (pGFP) to cells.

*Cellular transfection with plasmid pGFP:* Cellular transfection was carried out using pGFP plasmid in HaCaT, hnDf, cos-7, A549 and HeLa cells. These cell lines were obtained from ATCC (Manassas, VA) and maintained in complete DMEM, containing 10% fetal bovine serum, 100units/ml penicillin, 100 µg/mL streptomycin, 0.1 mM MEM non-essential amino acids (NEAA), 2 mM L-glutamine, at 37 °C in 5% CO₂ atmosphere.

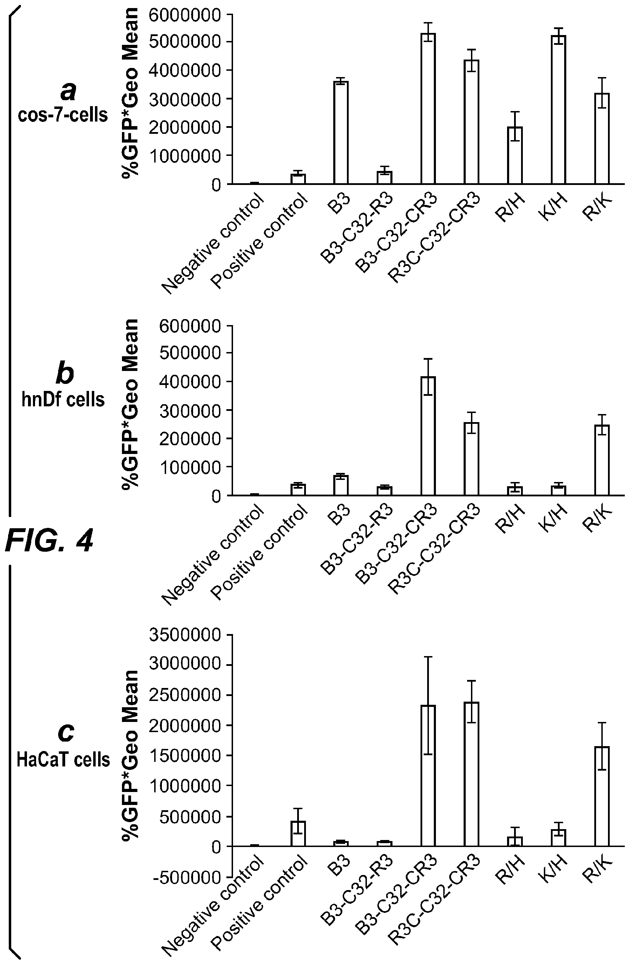

Cells were seeded on 96-well plates at 10,000 cells/well and incubated overnight to roughly 80-90% confluence prior to performing the transfection experiments. Polymer-DNA complexes were prepared as described above using the pGFP plasmid at a polymer:plasmid ratio of 50:1. Polyplexes were diluted in serum-free medium and added to cells at a final plasmid concentration of 0.6 µg pGFP/well. Cells were incubated for 3 h at 37°C in 5% CO₂ atmosphere. Subsequently, cells were washed once with PBS and complete DMEM was added. After 48h, cells were harvested and analysed for GFP expression by flow cytometry. GFP expression was compared against a negative control (untreated cells) and GeneJuice® (Merck KGaA, Germany) and B3-C32-B3 as a positive control. The results are shown in Figure 4a-c, wherein R/H, K/H and R/K represent 1:1 mixtures (w/w) of R3C-C32-CR3, K3C-C32-CK3 or H3C-C32-CH3 PBAEs.

### Example 11: Cytotoxicity

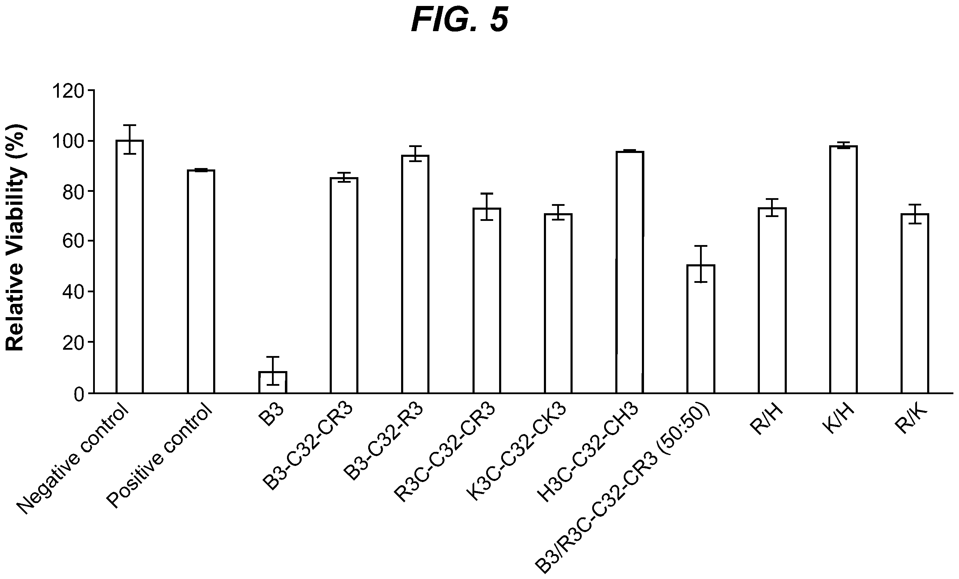

MTS assay (CellTiter 96® AQueous One Solution Cell Proliferation Assay, Promega Corporation, USA) was used to evaluate the viability of cos-7 cells transfected with the polymers described in the present application. Cell viability was assessed 48h after transfection using the MTS assay as instructed by the manufacturer. Briefly, cells were transfected with pGFP by a similar method to that in example 5. At 48h after transfection, the medium was removed, cells were washed with PBS and complete medium supplemented with MTS (20% v/v) was added. Cells were incubated at 37°C and absorbance was measured at 490 nm using a microplate reader. Cell viability was expressed as relative percentage compared to untreated cells. The results are shown in Figure 5, in which R/H, K/H and R/K represent 1:1 mixtures (w/w) of R3C-C32-CR3, K3C-C32-CK3 or H3C-C32-CH3 PBAEs.

### Example 12: Gene-silencing assay

The siRNA delivery efficiency of polymers of the present invention was assessed using GFP-specific siRNA in the GFP reporter stable cell line.

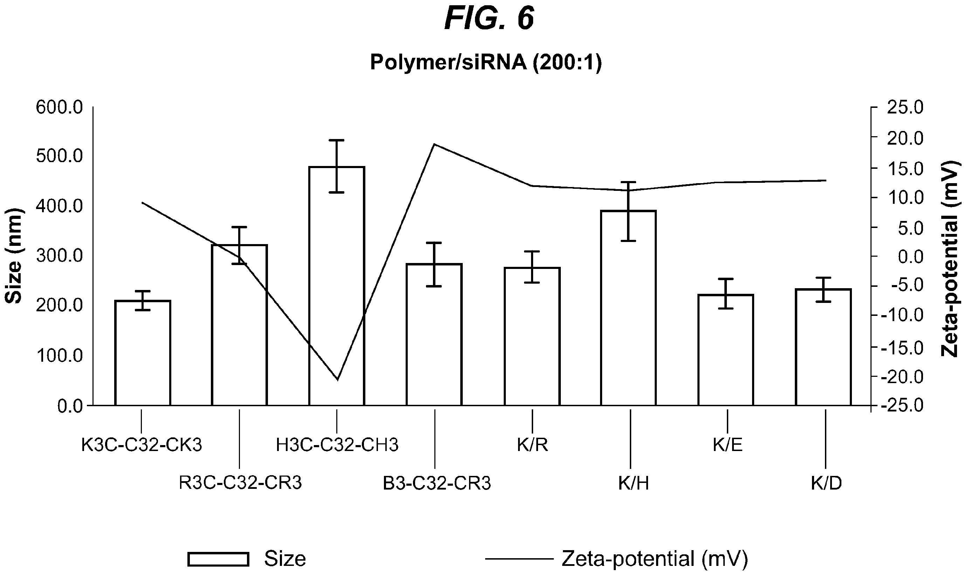

*Preparation of polymer-siRNA complexes:* Stock solutions of polymers were prepared in DMSO (100 mg/ml). These polymer solutions were diluted (25 mM acetate buffer pH 5.0) at appropriate concentration to obtain the desired ratio polymer-siRNA (w/w). Then 100 µl of appropriately diluted polymer was added to 100 µl of GFP-specific siRNA (10 µg/mL in acetate buffer 25 mM pH 5.0; ThermoScientific Dharmacon GFP Duplex I), mixed with vortex vigorously for few seconds then incubated at 37°C for 30 min. The resulting complexes were diluted in phosphate buffered saline for nanoparticle characterization. Polymer-siRNA complexes were characterized in terms of size and zeta potential using dynamic light scattering (Zetasizer nano zs90, Malvern Instruments). The results are shown in Figure 6, in which K/H and K/R represent 60:40 mixtures (w/w) of R3C-C32-CR3, K3C-C32-CK3 or H3C-C32-CH3 PBAEs. K/E and K/D represent 70:30 mixtures (w/w) of K3C-C32-CK3 and D3C-C32-CD3 or E3C-C32-CE3 PBAEs.

Cell transfection with GFP-specific siRNA: Cellular transfection was carried out using GFP-specific siRNA in MDA-MB-231/GFP cells (Cell Biolabs Inc.). Cells were maintained in complete DMEM, containing 10% fetal bovine serum, 100 units/ml penicillin, 100 µg/mL streptomycin, at 37°C in 5% CO₂ atmosphere.

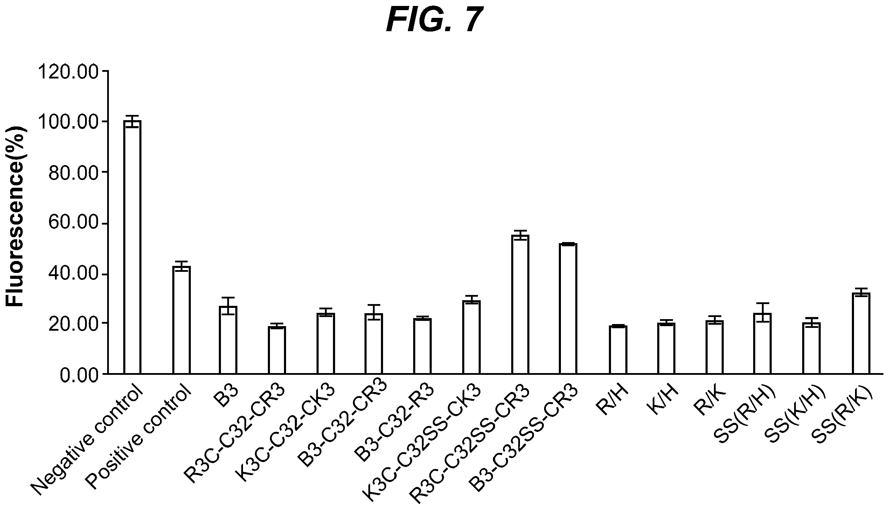

Briefly, cells were seeded on 96-well plates at 10,000 cells/well and incubated overnight to roughly 80-90% confluence prior to performing the transfection experiments. Polymer-siRNA complexes were prepared as described above using GFP-specific siRNA at a polymer:siRNA ratio of 200:1. Polyplexes were diluted in serum-free medium and added to cells at a final plasmid concentration of 50 nM siRNA/well. Cells were incubated for 3 h at 37 °C in 5% CO₂ atmosphere. Subsequently, cells were washed once with PBS and complete DMEM was added. After 48h, cells were harvested and analysed for GFP silencing by flow cytometry. Silencing in GFP expression was compared against a negative control (untreated cells) and INTERFERinTM (PolyPlus TransfectionTM) and B3 as positive controls. Results are shown in Figure 7, in which R/H, K/H and R/K represent 1:1 mixtures (w/w) of R3C-C32-CR3, K3C-C32-CK3 or H3C-C32-CH3 PBAEs, and SS(R/H), SS(K/H) and SS(R/K) represent 1:1 mixtures (w/w) of R3C-C32SS-CR3, K3C-C32SS-CK3 or H3C-C32SS-CH3 PBAEs.

### Example 13: Encapsulation of bovine insulin using glutamic acid and lysine end-modified PBAEs

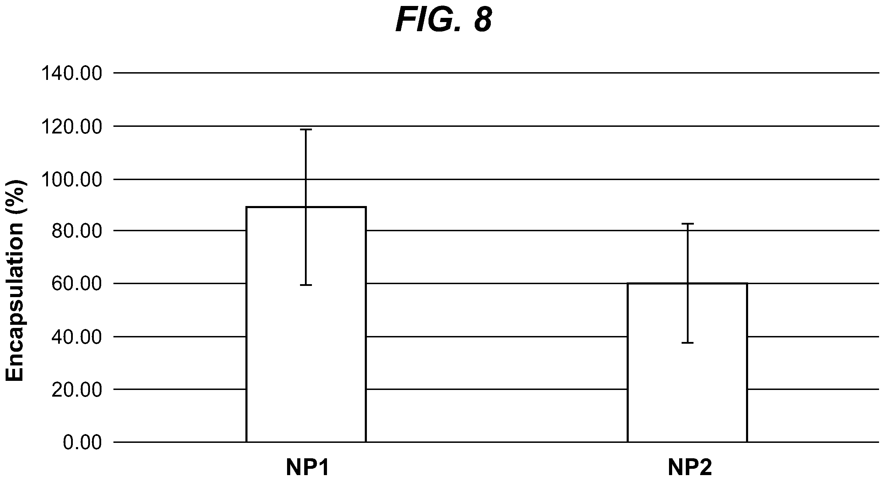

The encapsulation efficiency of polymers of the present invention was assessed using bovine insulin (Sigma Aldrich). Briefly, glutamic acid end-modified PBAEs E3C-C32-CE3 (16.7 µL at 60 mg/mL) was added to a solution of bovine insulin (1 mL at 0.01 mg/mL in HEPES buffer, 100 mM and pH 7.2) followed by lysine end-modified PBAEs K3C-C32-CK3 (10 µL at 100 mg/mL) to achieve a final polymer:protein ratio of 200:1. The mixture was incubated for 30 min at room temperature. The resulting nanoparticles were centrifuged using a Centricon device (10KDa cutoff, Merck Millipore) in order to separate insulin-containing nanoparticles from unencapsulated insulin. The degree of encapsulation was calculated by determining the unencapsulated insulin using the bicinchoninic assay (BCA protein assay reagent, ThermoScientific) and comparing with the original solution of insulin. The results are shown in Figure 8, in which NP1 and NP2 are independent duplicates.

## Claims

1. A polymer of formula I: 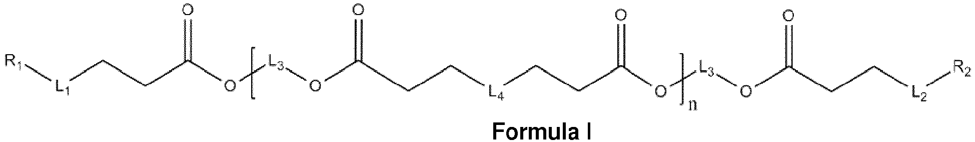 wherein
each L₁ and L₂ is independently selected from the group consisting of 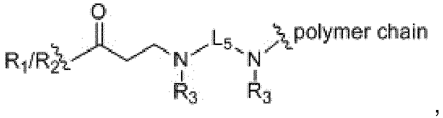 O, S, NRₓ and a bond; wherein Rₓ is independently selected from the group consisting of hydrogen, halogen, alkyl, cycloalkyl, alkenyl, cycloalkenyl, heteroalkyl, heterocycloalkyl, acyl, aryl or heteroaryl;
L₃ is independently selected from the group consisting of alkylene, alkenylene, heteroalkylene, heteroalkenylene, arylene or heteroarylene;
L₄ is selected from the group consisting of 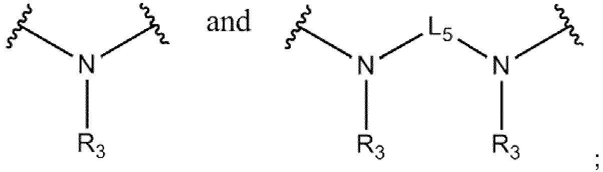
L₅ is independently selected from the group consisting of alkylene, alkenylene, heteroalkylene, heteroalkenylene, arylene or heteroarylene;
R₁ and R₂ are independently selected from an oligopeptide and R_{y};
wherein at least one of R₁ and R₂ is an oligopeptide;
wherein the or each oligopeptide has a net positive charge at pH 7; or wherein the or each oligopeptide comprises a mixture of naturally occurring amino acids that are negatively charged at pH 7 and naturally occurring amino acids that are positively charged at pH 7;
and wherein R_{y} is selected from the group consisting of hydrogen, halogen, alkyl, cycloalkyl, alkenyl, cycloalkenyl, heteroalkyl, heterocycloalkyl, acyl, aryl or heteroaryl; each R₃ is independently selected from the group consisting of hydrogen, halogen, alkyl, cycloalkyl, alkenyl, cycloalkenyl, heteroalkyl, heterocycloalkyl, acyl, aryl or heteroaryl; and n is an integer from 5 to 10,000;
or a pharmaceutically acceptable salt thereof.

2. The polymer of claim 1, wherein the or each oligopeptide comprises amino acid residues selected from the group consisting of lysine and arginine; or wherein the or each oligopeptide comprises a mixture of naturally occurring amino acids that are negatively charged at pH7 and naturally occurring amino acids that are positively charged at pH 7.

3. The polymer of claim 1 or claim 2, wherein the or each oligopeptide comprises from 3 to 20 amino acid residues.

4. The polymer of any preceding claim, wherein the or each oligopeptide has a net positive charge at pH 7.

5. The polymer of claim 1 or claim 2, wherein the or each oligopeptide comprises amino acid residues selected from the group consisting of lysine, arginine and histidine.

6. The polymer of any preceding claim, wherein the or each oligopeptide is a compound of **Formula VII:** 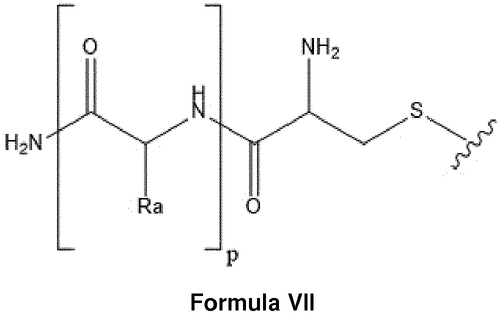 wherein p is an integer from 2 to 19 and wherein Rₐ is selected at each occurrence from the group consisting of H₂NC(=NH)-NH(CH₂)₃-, H₂N(CH₂)₄- or (1*H*-imidazol-4-yl)-CH₂-.

7. The polymer of any preceding claim, wherein R₁ and R₂ are both oligopeptides.

8. The polymer of claim 7, wherein R₁ and R₂ are different oligopeptides.

9. The polymer of any of claims 1-7, wherein one of R₁ and R₂ is an oligopeptide and one of R₁ and R₂ is R_{y}.

10. The polymer of any preceding claim, wherein n is from 1 to 20.

11. The polymer of any preceding claim, wherein R_{y} is selected from a group consisting of hydrogen, -(CH₂)ₘNH₂, -(CH₂)ₘNHMe, -(CH₂)ₘOH, -(CH₂)ₘCH₃, - (CH₂)₂(OCH₂CH₂)ₘNH₂, -(CH₂)₂(OCH₂CH₂)ₘOH or -(CH₂)₂(OCH₂CH₂)ₘCH₃ wherein m is an integer from 1 to 20.

12. The polymer of any preceding claim, wherein each L₃ is independently selected from the group consisting of -C₁₋₁₀ alkylene- (S-S)_{q}-C₁₋₁₀ alkylene-, wherein q is 0 or 1.

13. The polymer of any preceding claim, wherein each R₃ is independently selected from hydrogen, C₁₋₆ alkyl, C₂₋₆alkenyl, C₂₋₆alkynyl, C₁₋₆ hydroxyalkyl, hydroxyl, C₁₋₆alkoxy, halogen, aryl, heterocyclic, heteroaryl, cyano, -O₂C-C₁₋₆alkyl, carbamoyl, -CO₂H, -CO₂-C₁-₆alkyl, C₁₋₆ alkylthioether, thiol, or ureido.

14. A nanoparticle comprising
(a) a polymer according to formula I 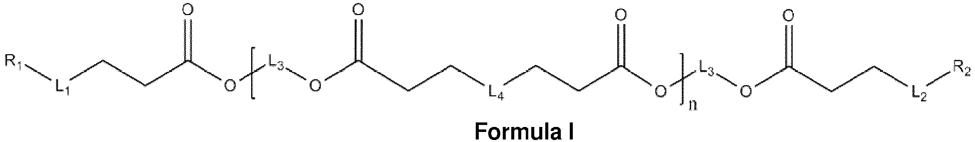 wherein
each L₁ and L₂ is independently selected from the group consisting of 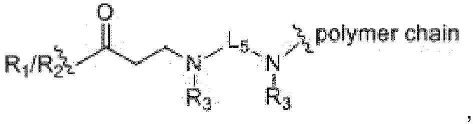
O, S, NRₓ and a bond; wherein Rₓ is independently selected from the group consisting of hydrogen, halogen, alkyl, cycloalkyl, alkenyl, cycloalkenyl, heteroalkyl, heterocycloalkyl, acyl, aryl or heteroaryl;
L₃ is independently selected from the group consisting of alkylene, alkenylene, heteroalkylene, heteroalkenylene, arylene or heteroarylene;
L₄ is selected from the group consisting of 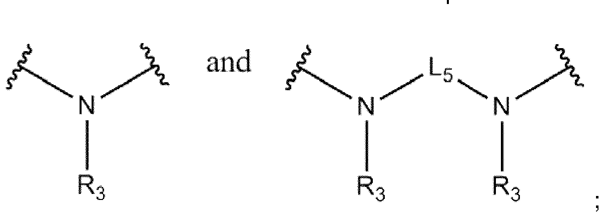
L₅ is independently selected from the group consisting of alkylene, alkenylene, heteroalkylene, heteroalkenylene, arylene or heteroarylene;
R₁ and R₂ are independently selected from an oligopeptide and R_{y};
wherein at least one of R₁ and R₂ is an oligopeptide;
wherein the or each oligopeptide has a net positive charge at pH 7;
and wherein R_{y} is selected from the group consisting of hydrogen, halogen, alkyl, cycloalkyl, alkenyl, cycloalkenyl, heteroalkyl, heterocycloalkyl, acyl, aryl or heteroaryl; each R₃ is independently selected from the group consisting of hydrogen, halogen, alkyl, cycloalkyl, alkenyl, cycloalkenyl, heteroalkyl, heterocycloalkyl, acyl, aryl or heteroaryl; and n is an integer from 5 to 10,000;
or a pharmaceutically acceptable salt thereof; and
(b) a polymer according to formula I 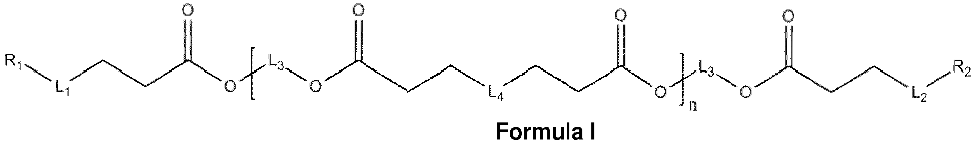 wherein
each L₁ and L₂ is independently selected from the group consisting of 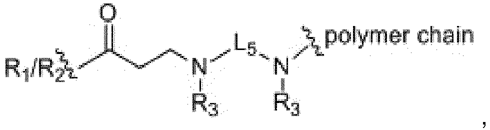
O, S, NRₓ and a bond; wherein Rₓ is independently selected from the group consisting of hydrogen, halogen, alkyl, cycloalkyl, alkenyl, cycloalkenyl, heteroalkyl, heterocycloalkyl, acyl, aryl or heteroaryl;
L₃ is independently selected from the group consisting of alkylene, alkenylene, heteroalkylene, heteroalkenylene, arylene or heteroarylene;
L₄ is selected from the group consisting of 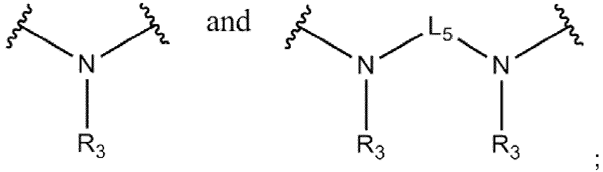
L₅ is independently selected from the group consisting of alkylene, alkenylene, heteroalkylene, heteroalkenylene, arylene or heteroarylene;
R₁ and R₂ are independently selected from an oligopeptide and R_{y};
wherein at least one of R₁ and R₂ is an oligopeptide;
wherein the or each oligopeptide has a net negative charge at pH 7;
and wherein R_{y} is selected from the group consisting of hydrogen, halogen, alkyl, cycloalkyl, alkenyl, cycloalkenyl, heteroalkyl, heterocycloalkyl, acyl, aryl or heteroaryl; each R₃ is independently selected from the group consisting of hydrogen, halogen, alkyl, cycloalkyl, alkenyl, cycloalkenyl, heteroalkyl, heterocycloalkyl, acyl, aryl or heteroaryl; and
n is an integer from 5 to 10,000;
or a pharmaceutically acceptable salt thereof.

15. A nanoparticle according to claim 14, further comprising an active agent.

16. A composition comprising an active agent and a polymer of any one of claims 1-13, or a composition comprising a nanoparticle according to claim 15.

17. The composition of claim 16, wherein the active agent is a polynucleotide.

18. The composition of claim 17, wherein the polynucleotide is RNA, DNA, or an siRNA.

19. The composition of claim 17 or 18, wherein the composition comprises nanoparticles containing the polynucleotide and the polymer.

20. A method of encapsulating an agent in a matrix of polymers according to any of claims 1 to 13 to form nanoparticles, the method comprising steps of: providing an agent; providing the polymer; and contacting the agent and the polymer under suitable conditions to form nanoparticles.

21. The method of claim 20, wherein the agent is a polynucleotide selected from DNA, RNA and siRNA, a small molecule or a protein.

22. The method of claim 20 or 21 wherein the step of contacting comprises (a) spray drying a mixture of the agent and the polymer, (b) double emulsion solvent evaporation techniques or (c) a phase inversion technique.

23. A polymer according to any one of claims 1 to 13, a nanoparticle according to claim 14 or claim 15, or a composition according to any one of claims 16 to 18 for use in medicine.

## Patentansprüche

1. Polymer der Formel I: 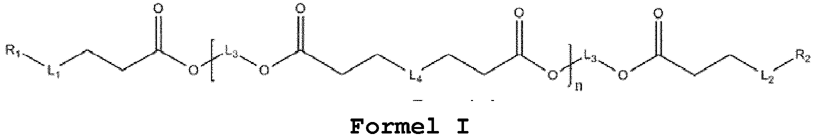 wobei
L₁ und L₂ jeweils unabhängig voneinander aus der Gruppe bestehend aus 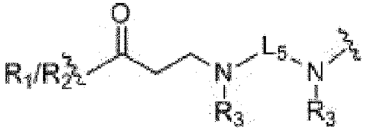 Polymerkette, O, S, NRₓ und einer Bindung ausgewählt sind; wobei Rₓ unabhängig aus der Gruppe bestehend aus Wasserstoff, Halogen, Alkyl, Cycloalkyl, Alkenyl, Cycloalkenyl, Heteroalkyl, Heterocycloalkyl, Acyl, Aryl oder Heteroaryl ausgewählt ist;
L₃ unabhängig aus der Gruppe bestehend aus Alkylen, Alkenylen, Heteroalkylen, Heteroalkenylen, Arylen oder Heteroarylen ausgewählt ist;
L₄ aus der Gruppe bestehend aus 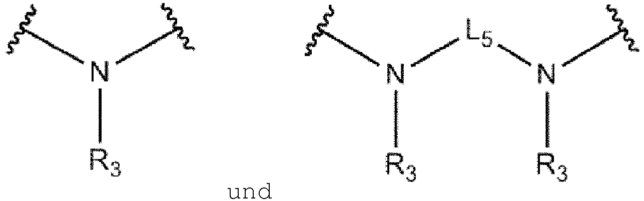 ausgewählt ist;
L⁵ unabhängig aus der Gruppe bestehend aus Alkylen, Alkenylen, Heteroalkylen, Heteroalkenylen, Arylen oder Heteroarylen ausgewählt ist;
R₁ und R₂ unabhängig aus einem Oligopeptid und R_{y} ausgewählt sind;
wobei mindestens eine der Variablen R₁ und R₂ für ein Oligopeptid steht;
wobei das bzw. jedes Oligopeptid bei pH 7 eine positive Nettoladung aufweist oder wobei das bzw. jedes Oligopeptid eine Mischung von natürlich vorkommenden Aminosäuren, die bei pH 7 negativ geladen sind, und natürlich vorkommenden Aminosäuren, die bei pH 7 positiv geladen sind, umfasst; und wobei R_{y} aus der Gruppe bestehend aus Wasserstoff, Halogen, Alkyl, Cycloalkyl, Alkenyl, Cycloalkenyl, Heteroalkyl, Heterocyclyl, Acyl, Aryl oder Heteroaryl ausgewählt ist;
R₃ jeweils unabhängig aus der Gruppe bestehend aus Wasserstoff, Halogen, Alkyl, Cycloalkyl, Alkenyl, Cycloalkenyl, Heteroalkyl, Heterocyclyl, Acyl, Aryl oder Heteroaryl ausgewählt ist; und
n für eine ganze Zahl von 5 bis 10.000 steht;
oder ein pharmazeutisch unbedenkliches Salz davon.

2. Polymer nach Anspruch 1, wobei das bzw. jedes Oligopeptid Aminosäurereste aus der Gruppe bestehend aus Lysin und Arginin umfasst oder wobei das bzw. jedes Oligopeptid eine Mischung von natürlich vorkommenden Aminosäuren, die bei pH 7 negativ geladen sind, und natürlich vorkommenden Aminosäuren, die bei pH 7 positiv geladen sind, umfasst.

3. Polymer nach Anspruch 1 oder Anspruch 2, wobei das bzw. jedes Oligopeptid 3 bis 20 Aminosäurereste umfasst.

4. Polymer nach einem der vorhergehenden Ansprüche, wobei das bzw. jedes Oligopeptid bei pH 7 eine positive Nettoladung aufweist.

5. Polymer nach Anspruch 1 oder Anspruch 2, wobei das bzw. jedes Oligopeptid Aminosäurereste aus der Gruppe bestehend aus Lysin, Arginin und Histidin umfasst.

6. Polymer nach einem der vorhergehenden Ansprüche, wobei es sich bei dem bzw. jedem Oligopeptid um eine Verbindung der Formel VII handelt: 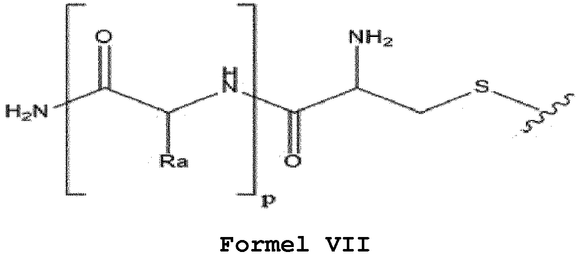 wobei p für eine ganze Zahl von 2 bis 19 steht und wobei Rₐ bei jedem Vorkommen aus der Gruppe bestehend aus H₂NC (=NH)-NH(CH₂)₃-, H₂N(CH₂)₄- oder (1*H-*Imidazol-4-yl)-CH₂-ausgewählt ist.

7. Polymer nach einem der vorhergehenden Ansprüche, wobei R₁ und R₂ beide für Oligopeptide stehen.

8. Polymer nach Anspruch 7, wobei R₁ und R₂ für verschiedene Oligopeptide stehen.

9. Polymer nach einem der Ansprüche 1-7, wobei eine der Variablen R₁ und R₂ für ein Oligopeptid steht und eine der Variablen R₁ und R₂ für R_{y} steht.

10. Polymer nach einem der vorhergehenden Ansprüche, wobei n für 1 bis 20 steht.

11. Polymer nach einem der vorhergehenden Ansprüche, wobei R_{y} aus der Gruppe bestehend aus Wasserstoff, - (CH₂)mNH₂, - (CH₂)ₘNHMe, -(CH₂)mOH, - (CH₂)ₘCH₃, -(CH₂)₂(OCH₂CH₂)ₘNH₂, -(CH₂)₂(OCH₂CH₂)ₘOH oder -(CH₂)₂(OCH₂CH₂)ₘCH₃ ausgewählt ist, wobei m für eine ganze Zahl von 1 bis 20 steht.

12. Polymer nach einem der vorhergehenden Ansprüche, wobei L₃ jeweils aus der Gruppe bestehend aus -C₁₋₁₀-Alkylen-(S-S)_{q}-C₁₋₁₀-alkylen-ausgewählt ist, wobei q für 0 oder 1 steht.

13. Polymer nach einem der vorhergehenden Ansprüche, wobei R₃ jeweils unabhängig aus Wasserstoff, C₁₋₆-Alkyl, C₂₋₆-Alkenyl, C₂₋₆-Alkinyl, C₁₋₆-Hydroxyalkyl, Hydroxyl, C₁₋₆-Alkoxy, Halogen, Aryl, Heterocyclyl, Heteroaryl, Cyano, -O₂C-C₁₋₆-Alkyl, Carbamoyl, -CO₂H, -CO₂-C₁₋₆-Alkyl, C₁₋₆-Alkylthioether, Thiol oder Ureido ausgewählt ist.

14. Nanopartikel, umfassend
(a) ein Polymer gemäß Formel I 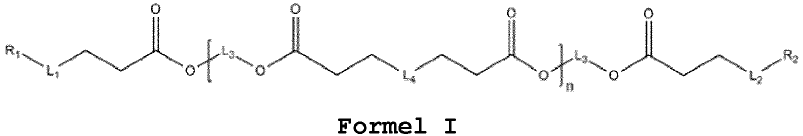 wobei
L₁ und L₂ jeweils unabhängig voneinander aus der Gruppe bestehend aus 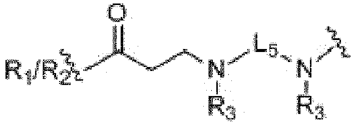 Polymerkette, O, S, NRₓ und einer Bindung ausgewählt sind; wobei Rₓ unabhängig aus der Gruppe bestehend aus Wasserstoff, Halogen, Alkyl, Cycloalkyl, Alkenyl, Cycloalkenyl, Heteroalkyl, Heterocycloalkyl, Acyl, Aryl oder Heteroaryl ausgewählt ist;
L₃ unabhängig aus der Gruppe bestehend aus Alkylen, Alkenylen, Heteroalkylen, Heteroalkenylen, Arylen oder Heteroarylen ausgewählt ist;
L₄ aus der Gruppe bestehend aus 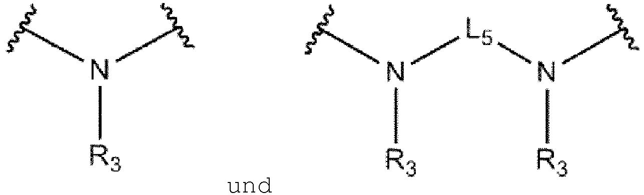 ausgewählt ist;
L⁵ unabhängig aus der Gruppe bestehend aus Alkylen, Alkenylen, Heteroalkylen, Heteroalkenylen, Arylen oder Heteroarylen ausgewählt ist;
R₁ und R₂ unabhängig aus einem Oligopeptid und R_{y} ausgewählt sind;
wobei mindestens eine der Variablen R₁ und R₂ für ein Oligopeptid steht;
woei das bzw. jedes Oligopeptid bei pH 7 eine positive Nettoladung aufweist;
und wobei R_{y} aus der Gruppe bestehend aus Wasserstoff, Halogen, Alkyl, Cycloalkyl, Alkenyl, Cycloalkenyl, Heteroalkyl, Heterocyclyl, Acyl, Aryl oder Heteroaryl ausgewählt ist;
R₃ jeweils unabhängig aus der Gruppe bestehend aus Wasserstoff, Halogen, Alkyl, Cycloalkyl, Alkenyl, Cycloalkenyl, Heteroalkyl, Heterocyclyl, Acyl, Aryl oder Heteroaryl ausgewählt ist; und
n für eine ganze Seite von 5 bis 10.000 steht; oder ein pharmazeutisch unbedenkliches Salz davon; und
(b) ein Polymer gemäß Formel I 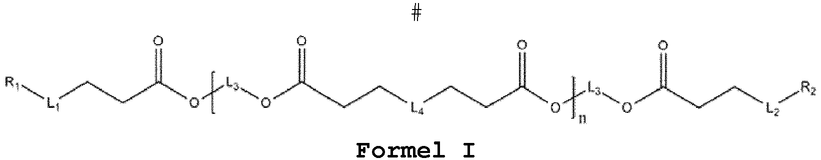 wobei
L₁ und L₂ jeweils unabhängig voneinander aus der Gruppe bestehend aus 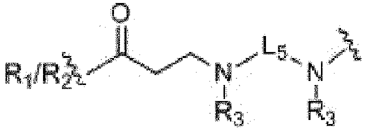 Polymerkette, O, S, NRₓ und einer Bindung ausgewählt sind; wobei Rₓ unabhängig aus der Gruppe bestehend aus Wasserstoff, Halogen, Alkyl, Cycloalkyl, Alkenyl, Cycloalkenyl, Heteroalkyl, Heterocycloalkyl, Acyl, Aryl oder Heteroaryl ausgewählt ist;
L₃ unabhängig aus der Gruppe bestehend aus Alkylen, Alkenylen, Heteroalkylen, Heteroalkenylen, Arylen oder Heteroarylen ausgewählt ist;
L₄ aus der Gruppe bestehend aus 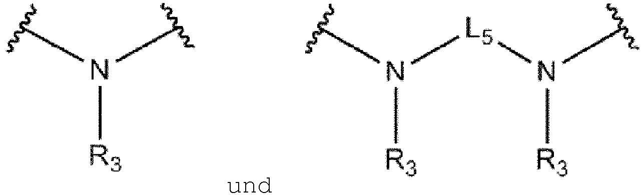 ausgewählt ist;
L⁵ unabhängig aus der Gruppe bestehend aus Alkylen, Alkenylen, Heteroalkylen, Heteroalkenylen, Arylen oder Heteroarylen ausgewählt ist;
R₁ und R₂ unabhängig aus einem Oligopeptid und R_{y} ausgewählt sind;
wobei mindestens eine der Variablen R₁ und R₂ für ein Oligopeptid steht;
wobei das bzw. jedes Oligopeptid bei pH 7 eine positive Nettoladung aufweist;
und wobei R_{y} aus der Gruppe bestehend aus Wasserstoff, Halogen, Alkyl, Cycloalkyl, Alkenyl, Cycloalkenyl, Heteroalkyl, Heterocyclyl, Acyl, Aryl oder Heteroaryl ausgewählt ist;
R₃ jeweils unabhängig aus der Gruppe bestehend aus Wasserstoff, Halogen, Alkyl, Cycloalkyl, Alkenyl, Cycloalkenyl, Heteroalkyl, Heterocyclyl, Acyl, Aryl oder Heteroaryl ausgewählt ist; und
n für eine ganze Zahl von 5 bis 10.000 steht;
oder ein pharmazeutisch unbedenkliches Salz davon.

15. Nanopartikel nach Anspruch 14, das ferner einen Wirkstoff umfasst.

16. Zusammensetzung, die einen Wirkstoff und ein Polymer nach einem der Ansprüche 1-13 umfasst, oder Zusammensetzung, die ein Nanopartikel nach Anspruch 15 umfasst.

17. Zusammensetzung nach Anspruch 16, wobei es sich bei dem Wirkstoff um ein Polynukleotid handelt.

18. Zusammensetzung nach Anspruch 17, wobei es sich bei dem Polynukleotid um RNA, DNA oder eine siRNA handelt.

19. Zusammensetzung nach Anspruch 17 oder 18, wobei die Zusammensetzung Nanopartikel, die das Polynukleotid und das Polymer enthalten, umfasst.

20. Verfahren zum Verkapseln eines Mittels in einer Matrix von Polymeren nach einem der Ansprüche 1 bis 13 zur Bildung von Nanopartikeln, wobei das Verfahren folgende Schritte umfasst: Bereitstellen eines Mittels; Bereitstellen des Polymers und Inkontaktbringen des Mittels und des Polymers unter geeigneten Bedingungen zur Bildung von Nanopartikeln.

21. Verfahren nach Anspruch 20, wobei es sich bei dem Mittel um ein Polynukleotid, das aus DNA, RNA und siRNA ausgewählt ist, ein kleines Molekül oder ein Protein handelt.

22. Verfahren nach Anspruch 20 oder 21, wobei der Schritt des Inkontaktbringens (a) das Sprühtrocknen einer Mischung des Mittels und des Polymers, (b) Doppelemulsionslösungsmittelverdampfungstechniken oder (c) eine Phaseninversionstechnik umfasst.

23. Polymer nach einem der Ansprüche 1 bis 13, Nanopartikel nach Anspruch 14 oder Anspruch 15 oder Zusammensetzung nach einem der Ansprüche 16 bis 18 zur Verwendung in der Medizin.

## Revendications

1. Polymère de formule I : 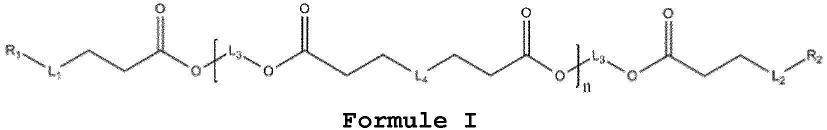 dans lequel
chaque L₁ et L₂ est indépendamment choisi dans le groupe constitué par 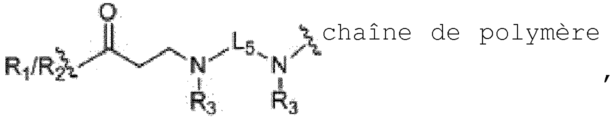
O, S, NRₓ et une liaison ; Rₓ étant indépendamment choisi dans le groupe constitué par l'atome d'hydrogène, les atomes d'halogène et les groupes alkyle, cycloalkyle, alcényle, cycloalcényle, hétéroalkyle, hétérocycloalkyle, acyle, aryle et hétéroaryle ;
L₃ est indépendamment choisi dans le groupe constitué par les groupes alkylène, alcénylène, hétéroalkylène, hétéroalcénylène, arylène et hétéroarylène ;
L₄ est choisi dans le groupe constitué par 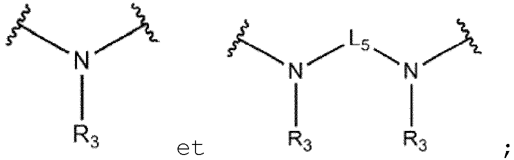
L₅ est indépendamment choisi dans le groupe constitué par les groupes alkylène, alcénylène, hétéroalkylène, hétéroalcénylène, arylène et hétéroarylène ;
R₁ et R₂ sont indépendamment choisis entre un oligopeptide et R_{y} ;
au moins l'un de R₁ et R₂ étant un oligopeptide ; l'oligopeptide ou chaque oligopeptide ayant une charge nette positive à pH 7 ; ou l'oligopeptide ou chaque oligopeptide comprenant un mélange d'acides aminés d'origine naturelle qui portent une charge négative à pH 7 et d'acides aminés d'origine naturelle qui portent une charge positive à pH 7 ;
et R_{y} étant choisi dans le groupe constitué par l'atome d'hydrogène, les atomes d'halogène et les groupes alkyle, cycloalkyle, alcényle, cycloalcényle, hétéroalkyle, hétérocycloalkyle, acyle, aryle et hétéroaryle ;
chaque R₃ est indépendamment choisi dans le groupe constitué par l'atome d'hydrogène, les atomes d'halogène et les groupes alkyle, cycloalkyle, alcényle, cycloalcényle, hétéroalkyle, hétérocycloalkyle, acyle, aryle et hétéroaryle ; et
n est un nombre entier de 5 à 10 000 ;
ou sel pharmaceutiquement acceptable de celui-ci.

2. Polymère selon la revendication 1, dans lequel l'oligopeptide ou chaque oligopeptide comprend des résidus d'acides aminés choisis dans le groupe constitué par la lysine et l'arginine ; ou dans lequel l'oligopeptide ou chaque oligopeptide comprend un mélange d'acides aminés d'origine naturelle qui portent une charge négative à pH 7 et d'acides aminés d'origine naturelle qui portent une charge positive à pH 7.

3. Polymère selon la revendication 1 ou la revendication 2, dans lequel l'oligopeptide ou chaque oligopeptide comprend de 3 à 20 résidus d'acides aminés.

4. Polymère selon une quelconque revendication précédente, dans lequel l'oligopeptide ou chaque oligopeptide a une charge nette positive à pH 7.

5. Polymère selon la revendication 1 ou la revendication 2, dans lequel l'oligopeptide ou chaque oligopeptide comprend des résidus d'acides aminés choisis dans le groupe constitué par la lysine, l'arginine et l'histidine.

6. Polymère selon une quelconque revendication précédente, dans lequel l'oligopeptide ou chaque oligopeptide est un composé de **formule VII :** 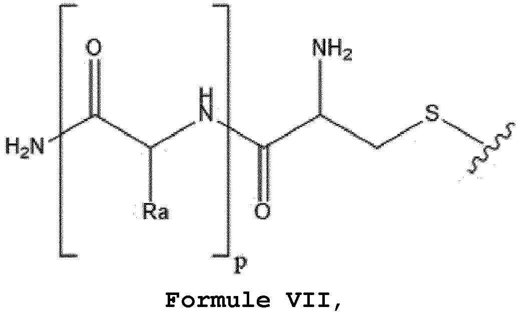 p étant un nombre entier de 2 à 19 et Rₐ étant choisi à chaque occurrence dans le groupe constitué par les groupes H₂NC(=NH)-NH(CH₂)₃-, H₂N(CH₂)₄- et (1*H*-imidazol-4-yl)-CH₂-.

7. Polymère selon une quelconque revendication précédente, dans lequel R₁ et R₂ sont tous deux des oligopeptides.

8. Polymère selon la revendication 7, dans lequel R₁ et R₂ sont des oligopeptides différents.

9. Polymère selon l'une quelconque des revendications 1-7, dans lequel l'un de R₁ et R₂ est un oligopeptide et l'un de R₁ et R₂ est R_{y}.

10. Polymère selon une quelconque revendication précédente, dans lequel n va de 1 à 20.

11. Polymère selon une quelconque revendication précédente, dans lequel R_{y} est choisi dans un groupe constitué par l'atome d'hydrogène et les groupes -(CH₂)ₘNH₂, -(CH₂)ₘNHMe, -(CH₂)ₘOH, -(CH₂)ₘCH₃, -(CH₂)₂(OCH₂CH₂)ₘNH₂, -(CH₂)₂(OCH₂CH₂)ₘOH et -(CH₂)₂(OCH₂CH₂)ₘCH₃, m étant un nombre entier de 1 à 20.

12. Polymère selon une quelconque revendication précédente, dans lequel chaque L₃ est indépendamment choisi dans le groupe constitué par les groupes -(alkylène en C₁₋₁₀)-(S-S)_{q}-(alkylène en C₁₋₁₀)-, q valant 0 ou 1.

13. Polymère selon une quelconque revendication précédente, dans lequel chaque R₃ est indépendamment choisi entre l'atome d'hydrogène et un groupe alkyle en C₁₋₆, alcényle en C₂₋₆, alcynyle en C₂₋₆, hydroxyalkyle en C₁₋₆, hydroxyle, alcoxy en C₁₋₆, halogéno, aryle, hétérocyclique, hétéroaryle, cyano, -O₂C-(alkyl en C₁₋₆), carbamoyle, -CO₂H, -CO₂- (alkyl en C₁₋₆), (alkyl en C₁₋₆)thioéther, thiol ou uréido.

14. Nanoparticule comprenant
(a) un polymère répondant à la formule I 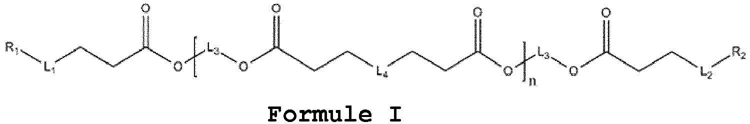 dans lequel
chaque L₁ et L₂ est indépendamment choisi dans le groupe constitué par 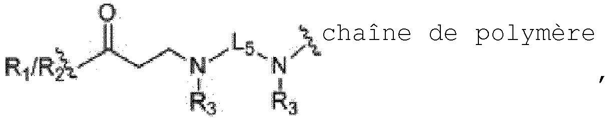
O, S, NRₓ et une liaison ; Rₓ étant indépendamment choisi dans le groupe constitué par l'atome d'hydrogène, les atomes d'halogène et les groupes alkyle, cycloalkyle, alcényle, cycloalcényle, hétéroalkyle, hétérocycloalkyle, acyle, aryle et hétéroaryle ;
L₃ est indépendamment choisi dans le groupe constitué par les groupes alkylène, alcénylène, hétéroalkylène, hétéroalcénylène, arylène et hétéroarylène ;
L₄ est choisi dans le groupe constitué par 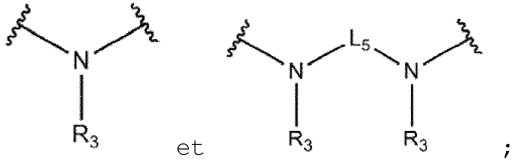
L₅ est indépendamment choisi dans le groupe constitué par les groupes alkylène, alcénylène, hétéroalkylène, hétéroalcénylène, arylène et hétéroarylène ;
R₁ et R₂ sont indépendamment choisis entre un oligopeptide et R_{y} ; au moins l'un de R₁ et R₂ étant un oligopeptide ; l'oligopeptide ou chaque oligopeptide ayant une charge nette positive à pH 7 ; et R_{y} étant choisi dans le groupe constitué par l'atome d'hydrogène, les atomes d'halogène et les groupes alkyle, cycloalkyle, alcényle, cycloalcényle, hétéroalkyle, hétérocycloalkyle, acyle, aryle et hétéroaryle ;
chaque R₃ est indépendamment choisi dans le groupe constitué par l'atome d'hydrogène, les atomes d'halogène et les groupes alkyle, cycloalkyle, alcényle, cycloalcényle, hétéroalkyle, hétérocycloalkyle, acyle, aryle et hétéroaryle ; et
n est un nombre entier de 5 à 10 000 ;
ou un sel pharmaceutiquement acceptable de celui-ci ; et
(b) un polymère répondant à la formule I 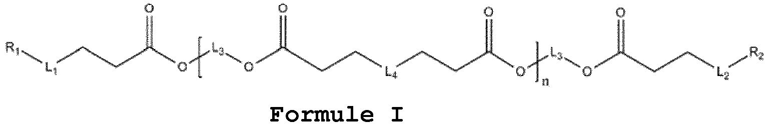 dans lequel
chaque L₁ et L₂ est indépendamment choisi dans le groupe constitué par 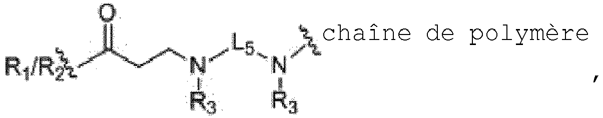
O, S, NRₓ et une liaison ; Rₓ étant indépendamment choisi dans le groupe constitué par l'atome d'hydrogène, les atomes d'halogène et les groupes alkyle, cycloalkyle, alcényle, cycloalcényle, hétéroalkyle, hétérocycloalkyle, acyle, aryle et hétéroaryle ;
L₃ est indépendamment choisi dans le groupe constitué par les groupes alkylène, alcénylène, hétéroalkylène, hétéroalcénylène, arylène et hétéroarylène ;
L₄ est choisi dans le groupe constitué par 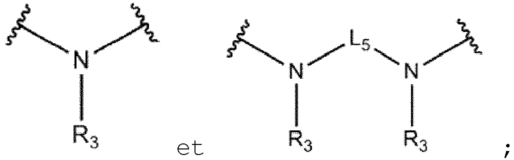
L₅ est indépendamment choisi dans le groupe constitué par les groupes alkylène, alcénylène, hétéroalkylène, hétéroalcénylène, arylène et hétéroarylène ;
R₁ et R₂ sont indépendamment choisis entre un oligopeptide et R_{y} ; au moins l'un de R₁ et R₂ étant un oligopeptide ; l'oligopeptide ou chaque oligopeptide ayant une charge nette négative à pH 7 ; et R_{y} étant choisi dans le groupe constitué par l'atome d'hydrogène, les atomes d'halogène et les groupes alkyle, cycloalkyle, alcényle, cycloalcényle, hétéroalkyle, hétérocycloalkyle, acyle, aryle et hétéroaryle ;
chaque R₃ est indépendamment choisi dans le groupe constitué par l'atome d'hydrogène, les atomes d'halogène et les groupes alkyle, cycloalkyle, alcényle, cycloalcényle, hétéroalkyle, hétérocycloalkyle, acyle, aryle et hétéroaryle ; et
n est un nombre entier de 5 à 10 000 ;
ou sel pharmaceutiquement acceptable de celui-ci.

15. Nanoparticule selon la revendication 14, comprenant en outre un agent actif.

16. Composition comprenant un agent actif et un polymère selon l'une quelconque des revendications 1-13 ou composition comprenant une nanoparticule selon la revendication 15.

17. Composition selon la revendication 16, dans laquelle l'agent actif est un polynucléotide.

18. Composition selon la revendication 17, dans laquelle le polynucléotide est de l'ARN, de l'ADN ou un ARNsi.

19. Composition selon la revendication 17 ou 18, la composition comprenant des nanoparticules contenant le polynucléotide et le polymère.

20. Procédé d'encapsulation d'un agent dans une matrice de polymères selon l'une quelconque des revendications 1 à 13 pour former des nanoparticules, le procédé comprenant les étapes de : fourniture d'un agent ; fourniture du polymère ; et mise en contact de l'agent et du polymère dans des conditions appropriées pour former des nanoparticules.

21. Procédé selon la revendication 20, dans lequel l'agent est un polynucléotide choisi entre de l'ADN, de l'ARN et de l'ARNsi, une petite molécule ou une protéine.

22. Procédé selon la revendication 20 ou 21 dans lequel l'étape de mise en contact comprend (a) le séchage par pulvérisation d'un mélange de l'agent et du polymère, (b) des techniques d'évaporation de solvant d'une émulsion double ou (c) une technique d'inversion de phases.

23. Polymère selon l'une quelconque des revendications 1 à 13, nanoparticule selon la revendication 14 ou la revendication 15 ou composition selon l'une quelconque des revendications 16 à 18 destinés à être utilisés en médecine.
